# EUROPEAN PATENT APPLICATION

(11) **EP 4 707 302 A2**
(43) Date of publication of application: **11.03.2026**
(21) Application number: 25215090.9
(22) Date of filing: 09.04.2021
(51) Int. Cl.: C07K 16/28

(54) **OFATUMUMAB FOR TREATING MS WHILE MAINTAINING SERUM IGG**

(30) Priority: 09.04.2020 EP 20169007; 22.05.2020 EP 20176057; 10.09.2020 US 202063076628 P; 25.03.2021 US 202163166054 P
(62) Divisional of application: 21716231.2
(71) Applicant: Novartis AG, 4056 Basel (CH)
(72) Inventor: PINGILI, Ratnakar, East Hanover, 07936-1080 (US); MERSCHHEMKE, Martin, 4056 Basel (CH)
(74) Representative: Carpmaels & Ransford LLP

(57) **Abstract**

The invention concerns ofatumumab for use in the treatment of multiple sclerosis (MS), wherein the treatment is a long-term treatment and wherein serum IgG level is maintained during the treatment. The invention further relates to B cell and/or T cell inhibitors for use in the treatment of MS, said treatment comprising monitoring serum IgG level and selecting ofatumumab as the B cell and/or T cell inhibitor if the serum IgG level decreases and/or said treatment comprising assessing the predisposition of the patient for an increased risk of infections and selecting ofatumumab as the B cell and/or T cell inhibitor if a predisposition is identified. The invention further relates to ofatumumab for use in the treatment of multiple sclerosis, wherein patients having a lowered serum IgG level are treated and/or wherein patients having risk factors associated with serum Ig levels, in particular serum IgG levels, are treated.

## Description

The invention concerns ofatumumab for use in the treatment of multiple sclerosis (MS), wherein the treatment is a long-term treatment and wherein the serum IgG level is maintained within a range that is close to untreated patients. The invention further relates to B cell and/or T cell inhibitors for use in the treatment of MS, said treatment comprising monitoring serum IgG levels and selecting ofatumumab as the B cell and/or T cell inhibitor if serum IgG level decreases and/or said treatment comprising assessing the predisposition of the patient for an increased risk of infections and selecting ofatumumab as the B cell and/or T cell inhibitor if a predisposition is identified. The invention further relates to ofatumumab for use in the treatment of multiple sclerosis, wherein patients having a lowered serum IgG level are treated and/or wherein patients having risk factors associated with serum Ig levels, in particular serum IgG levels, are treated.

Multiple Sclerosis (MS) is a chronic, immune-mediated disease of the central nervous system characterized by inflammation, demyelination and axonal/neuronal destruction, ultimately leading to severe disability. Although there is no cure for the disease, a variety of disease-modifying therapies (DMT) are available which usually slow down diseases progression.

Although most disease-modifying therapies for MS have traditionally been conceptualized as functioning via T cell-based mechanisms, a growing body of data indicates that these DMTs have demonstrable effects on B cells as well. Common themes include promoting naïve B cells rather than memory or plasmablasts (alemtuzumab); shifting B-cell cytokines towards an anti-inflammatory tone (beta interferon, glatiramer acetate, fingolimod); increasing B-regs (beta interferon, glatiramer acetate, fingolimod and dimethyl fumarate); decreasing class II MHC expression and costimulatory molecules on B cells required for antigen presentation (beta interferon and dimethyl fumarate); sequestering B cells in lymphoid organs (fingolimod); blocking VLA-4 mediated B cell trafficking to the CNS (natalizumab); or direct cytolysis of B cells (alemtuzumab, teriflunomide, mitoxantrone), see Greenfield et al., Ann Neurol. 2018 January; 83(1): 13-26.

Greenfield further reports that the monoclonal antibodies (mAbs) rituximab, ocrelizumab and ofatumumab which are each anti-CD20 antibodies are currently in clinical use for MS.

Rituximab, a chimeric mouse-human monoclonal antibody, was approved in 1997 for B cell lymphoma and indeed was one of the first mAbs ever developed for clinical use. Rituximab works primarily by depleting B cells through complement-dependent cytotoxicity (CDC), but also has significant antibody-dependent cellular cytotoxicity (ADCC) activity.

Ocrelizumab, now approved for relapsing and primary progressive forms of MS, differs from rituximab in that it has a humanized antibody backbone. Ocrelizumab exhibits greater ADCC compared to CDC than rituximab and also depletes B cells through multiple mechanisms, including apoptosis and antibody-dependent cellular phagocytosis.

Ofatumumab, a fully human monoclonal antibody approved for refractory chronic lymphocytic leukemia, results in greater CDC than ADCC activity and is the only anti-CD20 mAb now being tested using a subcutaneous, rather than intravenous, dosing regimen.

Other anti-CD20 mAbs include obinutuzumab, a humanized IgG1 targeting partly the same epitope of CD20 as rituximab, but designed to induce greater cell death due to its on/off binding rates, and ublituximab, an anti-CD20 antibody glycoengineered for higher affinity to all Fcγ RIIIa receptors, yielding greater ADCC than rituximab and ofatumumab, especially in cells with low CD20 expression.

However, treatment with B cell-depleting therapies has been reported to result in decreased serum levels of immunoglobulin IgG, IgM and/or IgA, see for example presentation of Dr. B. Wildemann at "8. Heidelberger Patiententag", January 25, 2020. Decreased IgG levels were reported to increase the risk of infections by a factor of three, decreased IgM levels were reported to double the risk of infections. In this regard it has to be kept in mind that MS therapy usually is a life-long therapy.

For example, under therapy with RTX (rituximab), patients experienced a significant time-dependent decrease of IgG and IgM levels in serum (IgG: *p*=2.2×10⁻⁵, IgM: *p*=4.0×10⁻⁴). Overall IgG levels decreased by 5.1% per year, IgM levels by 5.0%, see Klein et al., ECTRIMS Online Library,09/13/19; 278658; P1618.

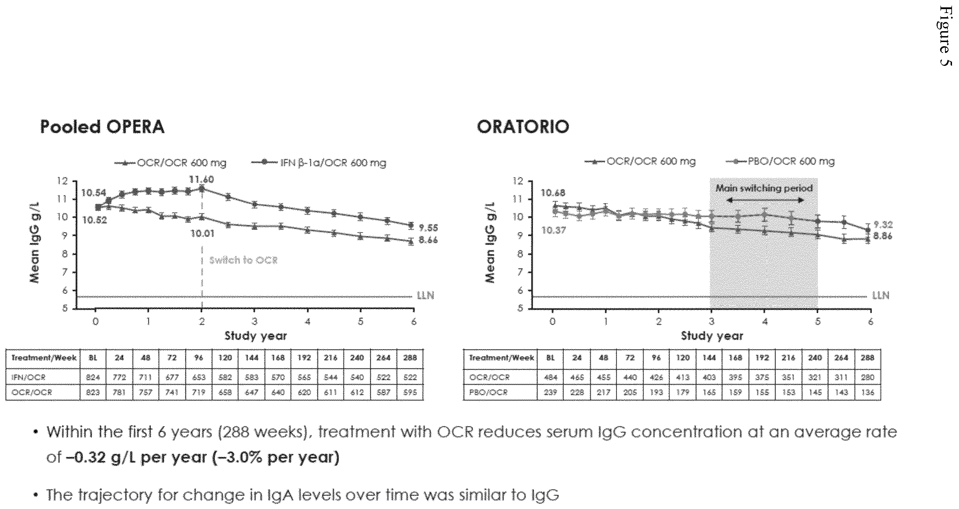

T. Derfuss et al. ("Serum immunoglobulin levels and risk of serious infections in the pivotal Phase III trials of ocrelizumab in multiple sclerosis and their open-label extensions", ECTRIMS Online Library. Derfuss T. 09/11/19; 279399; 65) assessed serum Ig levels over 5.5 years. They observed a reduction in serum Ig levels, with an apparent association with increased rates of serious infections. The association was strongest for IgG and less so for IgM or IgA. The reduction in serum Ig levels proceeded at an approximate mean rate of 3-4% per year (see Fig. 5). There was an apparent association between decreased levels of IgG and serious infections.

Further, the ocrelizumab prescribing information describes the association between the decrease in immunoglobulins and serious infections as follows: "Treatment with Ocrevus resulted in a decrease in total immunoglobulins over the controlled period of the studies mainly driven by reduction in IgM. Clinical trial data have shown an association between decreased levels of IgG (and less so for IgM or IgA) and serious infections."

Taken together, one of the most common adverse events associated with B cell-depleting therapies such as ocrelizumab therapy in clinical trials was reported to be a reduction of immunoglobulins (e.g. IgG) in the blood serum. In the long term, reduction of serum immunoglobulin levels is critical and not desirable.

Therefore, the problem underlying the present invention is to provide improved treatment strategies for MS patients, especially for long-term treatments. In particular, it was an object of the present invention to provide a B cell-depleting MS therapy without undesirably influencing serum levels of immunoglobulins.

The problem has unexpectedly been solved by the administration of ofatumumab.

It is completely surprising that ofatumumab therapy is advantageous compared to other B cell-depleting therapies because it does not cause reduction of immunoglobulins (e.g. IgG) in the extent known from the prior art. Consequently, the absence or amelioration of negative effects on the immune system opens up new avenues for patients under long-term treatment. This is an important clinical benefit which is explained in detail hereinafter.

Hence, a subject of the present invention relates to an B cell and/or T cell inhibitor for use in the treatment of multiple sclerosis, said treatment comprising
(a) optionally administering a B cell and/or T cell inhibitor, preferably other than ofatumumab,
(b) monitoring serum Ig levels, in particular the serum IgG level,
(c) selecting ofatumumab as the B cell and/or T cell inhibitor if the serum IgG level decreases; and/or
said treatment comprising
(a) optionally administering an B cell and/or T cell inhibitor, preferably other than ofatumumab,
(b) assessing the predisposition of the patient for an increased risk of infections,
(c) selecting ofatumumab as the B cell and/or T cell inhibitor if a predisposition for an increased risk of infections is identified.

A further subject of the present invention is ofatumumab for use in the treatment of multiple sclerosis, wherein the treatment is a long-term treatment and wherein the serum IgG level is maintained within a range, wherein said range is essentially the same in untreated patients.

In the context of the present invention, "untreated patients" refers to patients diagnosed with MS or clinically isolated syndrome (CIS) and which are not administered a B cell and/or T cell inhibitor. In a preferred embodiment the untreated patients present IgG levels in a range from 500 to 1800 mg/dl, particularly from 700 to 1600 mg/dl, more particularly from 900 to 1400 mg/dl. Particularly, the untreated patients present IgG levels from 500 mg/dl, 550 mg/dl, 600 mg/dl, 650 mg/dl, 700 mg/dl, 750 mg/dl, 800 mg/dl, 850 mg/dl, 900 mg/dl up to 1400 mg/dl, 1500 mg/dl, 1600 mg/dl, 1700 mg/dl, 1800 mg/dl.

Another subject of the present invention is ofatumumab for use in the treatment of multiple sclerosis, wherein patients having a lowered serum IgG level are treated.

Still another subject of the present invention is ofatumumab for use in the treatment of multiple sclerosis, wherein patients having risk factors associated with serum Ig levels, in particular serum IgG levels, are treated.

In an embodiment of the invention, ofatumumab is not administered to patients having an active HBV infection, particularly having an active HBV infection confirmed by positive results for Hepatitis B surface antigen [HBsAg] and anti-HBV tests. Ofatumumab may or may not be administered to patients who are negative for HBsAg and positive for Hepatitis B core antibody [HBcAb+] or are carriers of HBV [HBsAg+].

### Description of Preferred Embodiments

Generally, the present invention concerns the treatment of multiple sclerosis. In a preferred embodiment the invention relates to the treatment of relapsing multiple sclerosis (RMS). In particular, the present invention relates to the treatment of relapsing remitting multiple sclerosis (RRMS). Alternatively, the present invention relates to the treatment of secondary progressive MS (SPMS). Further alternatively, the present invention relates to the treatment of clinically isolated syndrome (CIS). Again further alternatively, the present invention relates to the treatment of primary progressive multiple sclerosis (PPMS) or progressive relapsing multiple sclerosis (PRMS).

Generally, in step (a) of the use of the present treatment, a B cell and/or T cell inhibitor other than ofatumumab can be administered.

Generally, the B cell and/or T cell inhibitor other than ofatumumab can be any drug inhibiting B cell and/or T cell activity and being suitable as a disease-modifying treatment (DMT) of multiple sclerosis. Such B cell and/or T cell inhibitor inhibiting B cell and/or T cell activity can result in depletion of B cells and/or T cells or may interfere otherwise with B cell and/or T cell activity. Examples of approved drugs resulting in a depletion of B cells are e.g. ocrelizumab, rituximamb, obinutuzumab and ublituximab.

Examples of other B cell and/or T cell inhibitors are e.g. cladribine, fingolimod, natalizumab, teriflunomide and the like.

In a preferred embodiment, the B cell and/or T cell inhibitor in step (a) is ocrelizumab or rituximab.

In a first aspect of the present invention, in step (b), at least one serum immunoglobulin (Ig) level is monitored.

In a preferred embodiment, IgG and IgM are monitored. In particular, IgG is monitored.

Generally, the monitoring step can be carried out by measuring the respective serum Ig levels, preferably on a regular basis. In a preferred embodiment serum Ig levels can be measured e.g. every month, every second month, every third month, every six months or every year. In a preferred embodiment the monitoring in step (b) is carried out every 1 - 12, in particular every 3 - 9 months. The IgG levels may be determined by the treating neurologist, e.g. during periodic visits

In a first aspect of the present invention, in step (c), ofatumumab is selected as the B cell and/or T cell inhibitor if the serum IgG level decreases. In other words, if serum IgG levels are undesirably low, treatment of MS is carried out or continued with ofatumumab as the DMT. In a preferred embodiment of the present invention, ofatumumab is administered in step (c) as the sole active ingredient for treating MS, i.e. the only disease-modifying drug that is administered.

According to the invention, "lowered serum IgG levels", "undesirably low serum IgG levels", "decreased serum IgG levels" and "decreasing serum IgG levels" refer to serum IgG levels falling below a concentration of 900 mg/dl, 850 mg/dl, 800 mg/dl, 750 mg/dl, 700 mg/dl, 650 mg/ml, 600 mg/dl, 550 mg/dl or 500 mg/dl and/or serum IgG levels falling below the lower limit of normal and/or serum IgG levels falling below 80%, below 70%, below 60% or below 50% of the baseline level at the start of DMT therapy.

In a preferred embodiment in step (c), ofatumumab is selected if the serum IgG level falls below a concentration of 900 mg/dl, 850 mg/dl, 800 mg/dl, 750 mg/dl, 700 mg/dl, 650 mg/ml, 600 mg/dl, 550 mg/dl or 500 mg/dl.

In a preferred embodiment, in step (c), ofatumumab is selected if the serum IgG level falls below the lower limit of normal, hereinafter referred to as "LLN".

Generally, the lower limit of normal (LLN) for IgG, IgA and IgM can be defined as IgG = 700 mg/dl or 565 mg/dl, IgM = 40 mg/dl and IgA = 70 mg/dl.

Consequently, serum IgG levels of < 700 mg/dl or < 565 mg/dl represent serum IgG levels below LLN, serum IgM levels of < 40 mg/dl represent serum IgM levels below LLN and serum IgA levels of < 70 mg/dl represent serum IgA levels below LLN. In a preferred embodiment, in step (c), ofatumumab is selected as the B cell and/or T cell inhibitor if the serum IgG level falls below 80%, below 70%, below 60% or below 50% of the baseline level at the start of DMT therapy. In other words, the serum Ig levels are measured at the start of DMT and set as baseline values, in particular the serum IgG level is measured at the start of DMT and set as a baseline value. Once the measured levels fall below said baseline value, particularly below 80%, below 70%, below 60% or below 50% of said baseline level, ofatumumab is selected as the DMT.

In a second aspect of the present invention, in step (b), the predisposition of the patient for risk of infections, in particular for an increased risk of infections is assessed. In a preferred embodiment, the predisposition for bacterial infections, fungal infections, viral infections is assessed.

Methods for the assessment of the predisposition of a patient for an increased risk of infections such as bacterial infections, fungal infections and viral infections are well known in the art. For the assessment of the predisposition of a patient for an increased risk of infections preferably immunoglobulin levels are indicative.

In a second aspect of the present invention, in step (c), ofatumumab is selected as the B cell and/or T cell inhibitor if a predisposition for an increased risk of infections is identified, in other words if the predisposition is increased. In a preferred embodiment of the present invention, ofatumumab is administered in step (c) as the sole active ingredient for treating MS, i.e. the only disease-modifying drug that is administered.

Another subject of the present invention is ofatumumab for use in the treatment or prevention of multiple sclerosis, wherein ofatumumab is used in a patient who has been treated with a B cell and/or T cell inhibitor, preferably other than ofatumumab. A further subject of the invention is a method for treating or preventing multiple sclerosis, wherein the method comprises administration of ofatumumab to a patient suffering from multiple sclerosis, said patient having taken a B cell and/or T cell inhibitor, preferably other than ofatumumab.

Generally, the B cell and/or T cell inhibitor other than ofatumumab can be regarded as an earlier disease modifying therapy (DMT). This means, in a preferred embodiment of the present invention the patient is switched from an earlier DMT to ofatumumab. Said therapy preferably comprising the administration of a B cell and/or T cell inhibitor other than ofatumumab. In other words, the invention concerns ofatumumab for use in the treatment or prevention of multiple sclerosis, wherein ofatumumab is used in a patient transitioning from a disease-modifying therapy, said therapy preferably comprising the administration of a B cell and/or T cell inhibitor other than ofatumumab.

In a preferred embodiment the patient is switched because the earlier DMT does not provide stable Ig, e.g. IgG, level. In particular, the patient is switched because the earlier DMT leads to decreasing Ig levels, e.g. IgG levels, especially during long-term treatment.

As mentioned above, a subject of the present invention is ofatumumab for use in the treatment of multiple sclerosis, wherein the treatment is a long-term treatment and wherein serum IgG level is maintained within a range, wherein said range is essentially the same in untreated patients.

In a preferred embodiment, the range of the serum IgG level is from 500 to 1800 mg/dl, particularly from 700 to 1600 mg/dl, more particularly from 900 to 1400 mg/dl. As mentioned above, a subject of the present invention is ofatumumab for use in the treatment of multiple sclerosis, wherein patients having a lowered serum IgG level are treated.

In a preferred embodiment, the lowered serum IgG level at the start of treatment is below 900 mg/dl, below 850 mg/dl, below 800 mg/dl, below 750 mg/dl, below 700 mg/dl, below 650 mg/dl, below 600 mg/dl, below 550 mg/dl or below 500 mg/dl. In another preferred embodiment, the lowered serum IgG at the start of treatment is below the lower limit of normal.

In an alternative preferred embodiment, the change in serum IgM levels from baseline from 100 to 600 mg/dl, preferably less than 500 mg/dl, less than 400 mg/dl, less than 300 mg/dl, less than 200 mg/dl or less than 100 mg/dl.

As mentioned above, a subject of the present invention is ofatumumab for use in the treatment of multiple sclerosis, wherein patients having risk factors associated with serum Ig levels, in particular serum IgG levels, are treated.

According to the present invention risk factors associated with serum Ig levels, in particular serum IgG levels may be metabolic abnormalities, such as obesity and/or metabolic syndrome; or habits, such as alcohol consumption and/or smoking.

In a preferred embodiment, the risk factors are related to drugs of abuse, in particular alcohol and/or nicotine.

In a preferred embodiment of the present invention, ofatumumab for use in treating MS is used in a long-term treatment. The term long-term treatment indicates that ofatumumab is used over an extended period of time. For example, ofatumumab can be used for more than 2 years, 3 years, 4 years, 5, years, 10 years. Ofatumumab might be used up to 5 years, 10 years, 15 years, 20 years or for life.

In a preferred embodiment of the present invention, ofatumumab is administered at a dose of 10 to 30 mg every 4 weeks, preferably 20 mg every 4 weeks.

Preferably, ofatumumab is administered parenterally, e.g. by epidermal, intravenous, intramuscular, intraarterial, intrathecal, intracapsular, intraorbital, intracardiac, intradermal, intraperitoneal, intratendinous, transtracheal, subcutaneous, subcuticular, intraarticular, subcapsular, subarachnoid, intraspinal, intracranial, intrathoracic, epidural or intrasternal injection or infusion. The preferred route of administration is a subcutaneous injection (sc).

In a preferred embodiment of the present invention, ofatumumab is administered with a loading dose. The term loading dose is defined below. In a preferred embodiment, three loading doses are administered, preferably in week 0 and in week 1 and in week 2 after starting ofatumumab therapy. This means the first loading dose in week 0 constitutes the start of therapy. In an alternative preferred embodiment, three loading doses are administered on day 1, on day 5 - 9, preferably on day 7, and on day 12 - 16, preferably on day 14, after starting ofatumumab therapy. This means the first loading dose on day 1 constitutes the start of therapy.

In a preferred embodiment of the present invention, a loading dose is 10 - 30 mg, preferably 20 mg ofatumumab.

In an alternative embodiment of the present invention, ofatumumab is administered without a loading dose.

In a preferred embodiment of the present invention, a premedication is administered to the patient before the first dose of ofatumumab is administered. Preferably, the premedication comprises a compound selected from acetaminophen, antihistamines and steroids. Methylprednisolone may be a preferred steroid. 100 mg iv may be a preferred dose. Preferably, the premedication is administered 30 to 60 minutes prior to an ofatumumab injection.

In a particular preferred embodiment, no premedication is administered prior to the first dose of ofatumumab.

In a preferred embodiment of the present invention, relapsing multiple sclerosis is either clinically isolated syndrome (CIS) or relapsing remitting multiple sclerosis (RRMS) or secondary progressive multiple sclerosis (SPMS). These terms are defined below.

In a preferred embodiment of the present invention, ofatumumab is administered as the sole active ingredient for treating MS. In other words, ofatumumab is preferably the only disease-modifying drug that is administered.

In a preferred embodiment, ofatumumab can be administered irrespective of body weight, sex, age, race or baseline B-cell count. For example, it is preferred that a 35-year-old woman having a body weight of 60 kg receives the same dose as a 50-year old man having a body weight of 90 kg. In particular, body weight, sex, age, race or baseline B-cell count do not have a clinically meaningful effect on the pharmacokinetics of ofatumumab.

In a preferred embodiment, ofatumumab is administered to patients who discontinued or interrupted, preferably discontinued earlier DMT, e.g. anti-CD20 therapy, because of side effects, e.g. side effects such as malignancies, severe infusion-related reactions, or recurrent infections.

For example, an increased number of malignancies (including breast cancers) have been observed in clinical trials in patients treated with ocrelizumab (Ocrevus^{®}), compared to control groups. Ocrevus' SmPC recommends that individual benefit/risk should be considered in patients who are being actively monitored for recurrence of malignancy. Patients with a known active malignancy should not be treated with Ocrevus.

As mentioned above, side effects and adverse events associated with B cell-depleting therapies such as ocrelizumab therapy are reported to be associated with a reduction of immunoglobulins (e.g. IgG). In the present invention it was surprisingly found that ofatumumab therapy is advantageous compared to other B cell-depleting therapies because it does not cause reduction of immunoglobulins (e.g. IgG) in the long run and thus opens up new avenues for patients under long-term treatment.

Hence, in a preferred embodiment of the present invention ofatumumab is used in the treatment of MS, wherein ofatumumab is administered to patients with known risk factors for malignancies. In another preferred embodiment of the present invention ofatumumab is used in the treatment of MS, wherein ofatumumab is administered to patients who are being actively monitored for recurrence of malignancy. In an alternative embodiment of the present invention ofatumumab is used in the treatment of MS, wherein ofatumumab is administered to patients with a known active malignancy.

The MSIS-29 (see definition below) is a clinically useful and scientifically sound measure of the impact of MS from the patient's perspective suitable for clinical studies and epidemiological studies. It is considered a reliable, valid and responsive PRO (Patient Reported Outcomes) measure that complements other indicators of disease severity used to improve our understanding of the impact of MS.

In the present invention, it was unexpectedly found that administration of ofatumumab leads to an advantageous reduction of the MS impact scale MSIS-29 as defined below. Reference is made to the experimental section below.

In this regard a further subject of the present invention is ofatumumab for use in the treatment or prevention of relapsing multiple sclerosis, wherein ofatumumab reduces the MSIS-29 score. Preferably, ofatumumab reduces the MSIS-29 score by at least 1.5, more preferably at least 2.0, still more preferably at least 2.5 within 24 months. The reduction might be up to 3.0 or 3.5 or 4.0.

In one embodiment of the invention, an ofatumumab composition is formulated in accordance with routine procedures as a pharmaceutical composition adapted for intravenous administration to human beings. Typically, compositions for intravenous administration are solutions in sterile isotonic aqueous buffer. Where suitable, the composition may also include a solubilizing agent and a local anesthetic, such as lignocaine, to ease pain at the injection site. Generally, the ingredients are supplied either separately or mixed together in a unit dosage form, for example as a dry lyophilized powder or water-free concentrate, in a hermetically sealed container, such as an ampoule or sachet, indicating the quantity of active agent.

Where the composition is to be administered by infusion, in particular by subcutaneous injection (s.c.), it can be dispensed with an infusion bottle containing sterile pharmaceutical grade water or saline.

Where the composition is administered by injection, an ampoule of sterile water for injection or saline can be provided so that the ingredients may be mixed prior to administration.

In one embodiment, a formulation for ofatumumab can be formulated according to a formulation disclosed in WO 2009/009407.

In one embodiment, ofatumumab is formulated in an antibody formulation wherein ofatumumab is present in an amount of about 20-300 mg/mL, 50-300 mg/mL, 100-300 mg/mL, 150-300 mg/mL, 200-300 mg/mL or 250-300 mg/mL, preferably at 50 mg/ml.

In one embodiment, ofatumumab is formulated in an antibody formulation wherein the formulation comprises 10 to 100 mM sodium acetate, 25 to 100 mM sodium chloride, 0.5 to 5% arginine free base, 0.02 to 0.2 mM EDTA, 0.01 to 0.2% polysorbate 80 and adjusted to pH 5.0 to 7.0. Preferably, the ofatumumab formulation comprises 50 mM sodium acetate, 51 mM sodium chloride, 1% arginine free base, 0.05 mM EDTA, 0.02% polysorbate 80 and adjusted to pH 5.5.

The preferred dosage of ofatumumab is:
- initial dosing of 20 mg by subcutaneous injection at Weeks 0, 1 and 2, followed by
- subsequent dosing of 20 mg by subcutaneous injection once monthly starting at Week 4.

If an injection of ofatumumab is missed, it should preferably be administered as soon as possible without waiting until the next scheduled dose. Subsequent doses should be administered at the recommended intervals.

In one embodiment, the ofatumumab formulation is provided in a pre-filled syringe or in an auto-injector, preferably a single-dose pre-filled syringe or a single-dose pre-filled auto-injector. Preferably, a pre-filled auto-injector designed for s.c. administration is used.

In a preferred embodiment, ofatumumab injection is a sterile, preservative-free solution for subcutaneous use. Preferably, each 20 mg/0.4 mL prefilled pen or prefilled syringe delivers 0.4 mL of solution. Preferably, each 0.4 mL contains 20 mg of ofatumumab and arginine (4 mg), disodium edetate (0.007 mg), polysorbate 80 (0.08 mg), sodium acetate trihydrate (2.722 mg), sodium chloride (1.192 mg) and Water for Injection, USP with a pH of 5.5. Hydrochloric acid may be added to adjust pH.

In a preferred embodiment the ofatumumab formulation is intended for patient self-administration, preferably by subcutaneous injection.

In a preferred embodiment said formulation is administered in the abdomen, thigh or outer upper arm subcutaneously. In a preferred embodiment said formulation is not administered into moles, scars or areas where the skin is tender, bruised, red, hard or not intact.

In an embodiment, the first injection of said ofatumumab formulation may be performed under the guidance of a healthcare professional. If injection-related reactions occur, symptomatic treatment is recommended. Before administration, the pen or prefilled syringe is preferably removed from the refrigerator and allowed to reach room temperature, e.g. for about 15 to 30 minutes. In a preferred embodiment, the ofatumumab formulation of the present invention is a clear to slightly opalescent and colorless to slightly brownish-yellow solution available as follows:
- Injection: 20 mg/0.4 mL in a single-dose prefilled pen, e.g. Sensoready^{®} pen
- Injection: 20 mg/0.4 mL in a single-dose prefilled syringe.

In a preferred embodiment, a subcutaneous ofatumumab dose of 20 mg every 4 weeks leads to a mean AUCₜₐᵤ of about 400 to 550, more preferably 450 to 500, e.g. 483 mcg h/mL and/or to a mean Cₘₐₓ of 1.0 to 2.5, more preferably 1.2 to 1.7, e.g. 1.43 mcg/mL at steady state. In a preferred embodiment, the volume of distribution at steady-state can be 4.5 to 6.5, more preferably 5.0 to 6.0, e.g. 5.42 L following subcutaneous administration of repeated ofatumumab 20 mg doses.

After subcutaneous administration, ofatumumab can be absorbed via the lymphatic system.

In a preferred embodiment of the invention, ofatumumab is administered for the treatment of relapsing forms of multiple sclerosis (MS), to include clinically isolated syndrome, relapsing-remitting disease and active secondary progressive disease, preferably in adults.

In a preferred embodiment of the invention, ofatumumab administration is delayed in patients with an active infection, e.g. COVID-19, until the infection is resolved. Alternatively, the ofatumumab can be administered during an infection, e.g. during a COVID-19 infection. Thus, ofatumumab administration can be continued during an infection, e.g. during a COVID-19 infection.

In another preferred embodiment of the invention, the level of immunoglobulins at the beginning, during and after discontinuation of treatment with ofatumumab are monitored as clinically indicated until B-cell repletion. Discontinuing ofatumumab treatment is considered if a patient develops a serious opportunistic infection or recurrent infections if immunoglobulin levels indicate immune compromise.

A further subject of the invention is a method for treating multiple sclerosis, said treatment comprising
(a) optionally administering a B cell and/or T cell inhibitor, preferably other than ofatumumab to a patient in need thereof,
(b) monitoring serum Ig levels, in particular serum IgG level,
(c) selecting and administering ofatumumab as the B cell and/or T cell inhibitor if the serum IgG level decreases; and/or
said treatment comprising
(a) optionally administering the B cell and/or T cell inhibitor, preferably other than ofatumumab to a patient in need thereof,
(b) assessing the predisposition of the patient for an increased risk of infections
(c) selecting and administering ofatumumab as the B cell and/or T cell inhibitor if a predisposition for an increased risk of infections is identified.

A further subject of the invention is a method for treating multiple sclerosis, said treatment comprising administering ofatumumab to a patient in need thereof, wherein the treatment is a long-term treatment and wherein serum IgG level is maintained during the treatment.

A further subject of the invention is a method for treating multiple sclerosis, said treatment comprising administering ofatumumab to a patient in need thereof, wherein patients having a lowered serum IgG level are treated.

A further subject of the invention is a method for treating multiple sclerosis, said treatment comprising administering ofatumumab to a patient in need thereof, wherein patients having risk factors associated with serum Ig levels, in particular serum IgG levels are treated.

A further subject of the present invention is a method for the manufacture of a medicament for use in the treatments described above.

### Definitions

The terms "treatment" or "treat" can be defined as the application or administration of e.g. ofatumumab to a patient, where the purpose is to abolish, reduce or alleviate the symptoms of a disease such as multiple sclerosis (MS). In particular, the term "treatment" comprises the achievement of a clinically meaningful effect for the patient, for example the achievement of a clinically meaningful reduction of the annual relapse rate when treating RMS.

As used herein, a patient can be "in need of" a treatment if such a patient would benefit medically or in terms of the quality of life from such treatment.

The term "patient" as used herein can be a mammal, e.g. a primate, preferably a higher primate, especially preferred a human (e.g. a patient having a risk or at risk of having a disorder described herein). Preferably, the patient is an adult. Generally, also geriatric patients are included, however, patients between 18 and 60 years of age are preferred. As used herein, the term "administering" or "administration" of ofatumumab can mean providing ofatumumab to a patient in need of treatment. Administration "in combination with" one or more further therapeutic agents includes simultaneous (concurrent) and consecutive administration in any order and in any route of administration.

As used herein, a "therapeutically effective amount" can refer to an amount of ofatumumab that is effective, i.e. achieves a clinically meaningful effect.

The term "adverse event" (AE) can relate to any untoward medical occurrence in a patient or clinical investigation wherein the subject is administered a pharmaceutical product which does not necessarily have a causal relationship with this treatment. An adverse event (AE) can therefore be any unfavourable and unintended sign (including an abnormal laboratory finding), symptom or disease temporally associated with the use of a medicinal (investigational) product, whether or not related to the medicinal (investigational) product.

The phrase "therapeutic regimen" can mean the regimen used to treat an illness or to prevent a disease condition or the development of a disease, e.g. the dosing used. A therapeutic regimen may include an induction regimen, a loading regimen and a maintenance regimen.

### RRMS

Relapsing-remitting multiple sclerosis (RRMS) may be characterized by relapses, defined as a new neurologic deficit or episode of neurologic worsening lasting longer than 24 h, preferably in the absence of fever or infection.

There may be no apparent progression of the disease during the periods of remission. At different points in time, RRMS might be further characterized as either active (with relapses and/or evidence of new MRI activity) or not active, as well as worsening (a confirmed increase in disability over a specified period of time following a relapse) or not worsening. Reference is made to Lublin, Neurology. 2014 Jul 15; 83(3): 278-286.

### RMS

The term RMS (relapsing multiple sclerosis) encompasses RRMS, SPMS and clinically isolated syndrome (CIS).

### Primary progressive MS (PPMS)

PPMS can be characterized by worsening neurologic functions (accumulation of disability) from the onset of symptoms, without early relapses or remissions. PPMS can be further characterized at different points in time as either active (with an occasional relapse and/or evidence of new MRI activity) or not active, as well as with progression (evidence of disease worsening on an objective measure of change over time, with or without relapse or new MRI activity) or without progression. References is made to Lublin 2014.

Each person's experience with PPMS will be unique. PPMS can have brief periods when the disease is stable, with or without a relapse or new MRI activity, as well as periods when increasing disability occurs with or without new relapses or lesions on MRI.

### Secondary progressive MS (SPMS)

SPMS follows an initial relapsing-remitting course. Most people who are diagnosed with RRMS will eventually transition to a secondary progressive course in which there is a progressive worsening of neurologic function (accumulation of disability) over time. SPMS can be further characterized at different points in time as either active (with relapses and/or evidence of new MRI activity) or not active, as well as with progression (evidence of disease worsening on an objective measure of change over time, with or without relapses) or without progression. Reference is made to Lublin 2014.

Each person's experience with SPMS will be unique. SPMS follows after relapsing-remitting MS. Disability gradually increases over time, with or without evidence of disease activity (relapses or changes on MRI). In SPMS, occasional relapses may occur, as well as periods of stability.

### Relapse

Relapses can be defined as a new neurologic deficit or episode of neurologic worsening, preferably lasting longer than 24 h. In other words, relapses can be regarded as discrete episodes (in the art also referred to as "attacks," "flare-ups" or "exacerbations") of neurologic dysfunction, preferably lasting at least 24 h. Usually, relapses are followed by full or partial recovery and a period in which there is no symptom progression or accumulation of disability (remission).

"B cell inhibitor" as used herein generally may relate to any substance that abolishes, reduces or attenuates biological B cell functions. The B cell inhibitor may interrupt signal transduction pathways that are necessary for biological B cell functions, e.g. cytokine secretion or responses to cis and/or trans stimulation. The B cell inhibitor may also interfere with the generation of B cells from stem/progenitor cells or negatively affect their maturation. Moreover, the B cell inhibitor may act by inhibiting the cross-talk with other cell populations such as T cells. Alternatively, the B cell inhibitor may deplete B cells by sequestration (e.g. into lymphoid tissues such as the spleen) or by lysis e.g. through CDC, ADCC, phagocytosis or other processes. Several subsets of B cells may express CD20.

B cell as used herein may relate to a type of white blood cell of the lymphocyte subtype. B cells function in the humoral immunity component of the adaptive immune system by secreting antibodies such as immunoglobulins (e.g. IgG). Additionally, B cells may present antigens and secrete cytokines. B cells, unlike the T cells and natural killer cells, express B cell receptors (BCRs) on their cell membrane. BCRs allow the B cell to bind to a specific antigen against which it will initiate an antibody response.

T cell inhibitor as used herein may relate to any substance that abolishes, reduces and/or attenuates biological T cell functions. The T cell inhibitor may interrupt signal transduction pathways that are necessary for biological T cell functions e.g. cytokine secretion or responses to cis and/or trans stimulation. The T cell inhibitor may also interfere with the generation of T cells from stem/progenitor cells or negatively affect their maturation. Moreover, the T cell inhibitor may act by inhibiting the cross talk with other cell populations such as B cells. Alternatively, the T cell inhibitor may deplete T cells by sequestration (e.g. into lymphoid tissues such as the spleen) or by lysis, e.g. through CDC, ADCC, phagocytosis or other processes.

T cell as used herein may relate to a type of lymphocyte which develops in the thymus gland. T cells can be distinguished from other lymphocytes by the presence of a T cell receptor on the cell surface.

### Clinically isolated syndrome (CIS):

Clinically isolated syndrome (CIS) may refer to a single clinical attack of central nervous system (CNS) inflammatory demyelinating symptoms that are suggestive of multiple sclerosis (MS). CIS presentations can be monofocal or multifocal and typically may involve the optic nerve, brainstem, cerebellum, spinal cord or cerebral hemispheres. Reference is made to Miller et al, Clinically isolated syndromes, Lancet Neurol. 2012;11:157-169.

### Multiple Sclerosis Impact Scale (MSIS-29)

The MSIS-29 version 2 is a 29-item, self-administered questionnaire that includes 2 domains: physical and psychological. Responses were captured on a 4-point ordinal scale ranging from 1 (not at all) to 4 (extremely), with higher scores reflecting greater impact on day-to-day life. The MSIS-29 takes about 5 minutes to complete and the questions are designed to determine the patient's views about the impact of MS on their day-to-day life during the past 2 weeks. Reference is made to Hobart J and Cano S (2009), "Improving the evaluation of therapeutic interventions in multiple sclerosis: the role of new psychometric methods", Health Technol Assess; 13(12):iii, ix-x, 1-177. NS RO to Hobart J, Lamping D, Fitzpatrick R, et al (2001), "The Multiple Sclerosis Impact Scale (MSIS-29): a new patient-based outcome measure", Brain; 124(Pt 5):962-73.

### Ofatumumab:

Ofatumumab is a human monoclonal antibody for the CD20 protein. Ofatumumab may bind specifically to both the small and large extracellular loops of the CD20 molecule. The Fab domain of ofatumumab may bind to the CD20 molecule and the Fc domain mediates immune effector functions to result in B-cell lysis in vitro. In particular, ofatumumab is a recombinant human monoclonal immunoglobulin G1 (IgG1) antibody that binds to human CD20 expressed on e.g. B cells. Ofatumumab is produced in a murine NS0 cell line and consists of two IgG1 heavy chains and two kappa light chains with a molecular weight of approximately 146 kDa.

Ofatumumab is described in EP 1 558 648 B1 and EP 3 284 753 B1. Further reference is made to the description in the drugbank.ca, accession number DB06650 and to WHO Drug Information, Vol. 20, No. 1, 2006. In an embodiment, the protein chemical formula is C₆₄₈₀H₁₀₀₂₂N₁₇₄₂O₂₀₂₀S₄₄ and the protein average weight is about 146100 Da. In the US ofatumumab is marketed under the tradename Kesimpta^{®}.

The metabolic pathway of ofatumumab can be degradation to small peptides and amino acids by ubiquitous proteolytic enzymes. Ofatumumab might be eliminated in two ways: a target-independent route as with other IgG molecules and a target-mediated route that is related to binding to B cells.

The half-life of ofatumumab at steady state can be approximately 16 days, in particular following subcutaneous administration of repeated 20 mg doses.

Ofatumumab preferably does not share a common clearance pathway with chemical drugs that are metabolized by the cytochrome P450 system or other drug metabolizing enzymes. Preferably, ofatumumab is not involved in the regulation of the expression of drug metabolizing enzymes.

### Patient

The term "patient" preferably refers to a human patient, preferably an adult.

### Loading dose

A loading dose is an initial dose of a drug, preferably an initial higher dose, that may be given at the beginning of a treatment (e.g. a DMT) before transitioning to a maintenance dose, preferably being lower than the loading dose.

Immunoglobulins (Ig) and the subtypes IgG, IgA, IgM, IgD and IgE are commonly known and e.g. described in Berg/Tymoczko/Stryer "Biochemie", 5th edition, pp. 1015-1018. The present application focuses on serum levels of IgG and IgM, in particular IgG.

Serum immunoglobulin (Ig) levels can be determined routinely in clinical practice. In the present application serum Ig levels can preferably be determined by immunoturbidimetry. Preferably a Roche cobas^{®} Analyzer was used, especially module c of the cobas^{®} analyser. In a preferred embodiment Ig levels can be determined by using a cobas^{®} c 311 analyzer. It is further preferred that IgG measurements are carried out as described in the cobas^{®} leaflet "IGG-2 Tina-quant IgG Gen.2", preferably version 11.0 dated 2016-02 and IgM measurements as described in the leaflet "IGM-2 Tina-quant IgM Gen.2", preferably version 13.0 dated 2018-11.

Serum samples were preferably kept at 2 - 8 °C before measurement.

### Description of Figures:

Figure 1 describes the set-up of the clinical trial according to example 1 and the measurements of IgG and IgM.
Figure 2 illustrates the change of serum IgG levels from baseline.
Figure 3 illustrates the change of serum IgM levels from baseline.
Figure 4 shows the proportion of patients with IgG level (at least once anytime during the post-baseline visit) < LLN and < 50 % of LLN.
Figure 5 shows the decrease in IgG levels following administration of an anti-CD20 antibody of the prior art (Ocrelizumab). Fig. 5 was first published as part of T. Derfuss et al.: "Serum immunoglobulin levels and risk of serious infections in the pivotal Phase III trials of ocrelizumab in multiple sclerosis and their open-label extensions", ECTRIMS Online Library. Derfuss T. 09/11/19; 279399; 65.
Figure 6 shows that, after two years (96 weeks), ocrevus treatment (pooled OPERA, see Fig. 1) has led to a reduction of IgG levels by approx. 5% whereas ofatumumab has led to an increase of about 3%.

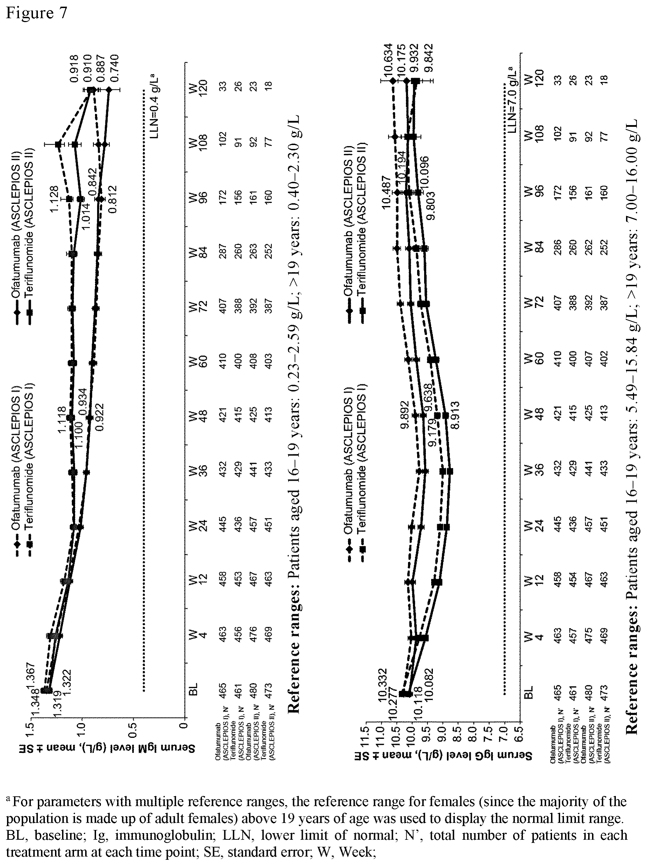

Figure 7 illustrates the change of serum IgM and IgG levels from baseline. Mean IgM and IgG levels remained well within the reference ranges over time. A reduction in IgM levels from baseline was observed in both treatment groups in both studies (ASCLEPIOS I and II). A reduction in IgG levels from baseline was observed until Week 36 and recovered thereafter in both treatment groups in both studies (ASCLEPIOS I and II). In ofatumumab-treated patients, the IgG levels recovered up to baseline levels by W72.

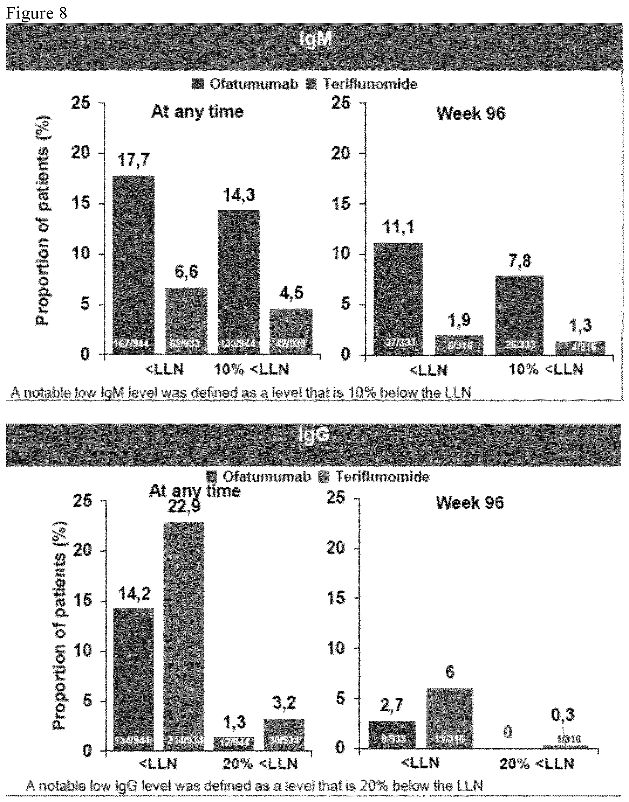

Figure 8 shows the proportion of patients with IgM and IgG levels < LLN, < 10% LLN, and < 20% LLN. At any time post baseline and at Week 96, a higher proportion of patients on ofatumumab had IgM levels below LLN and 10% below the LLN versus teriflunomide. At any time post-baseline, a lower proportion of patients on ofatumumab had IgG levels below the LLN versus teriflunomide. At Week 96, no ofatumumab patients had IgG levels 20% below the LLN.

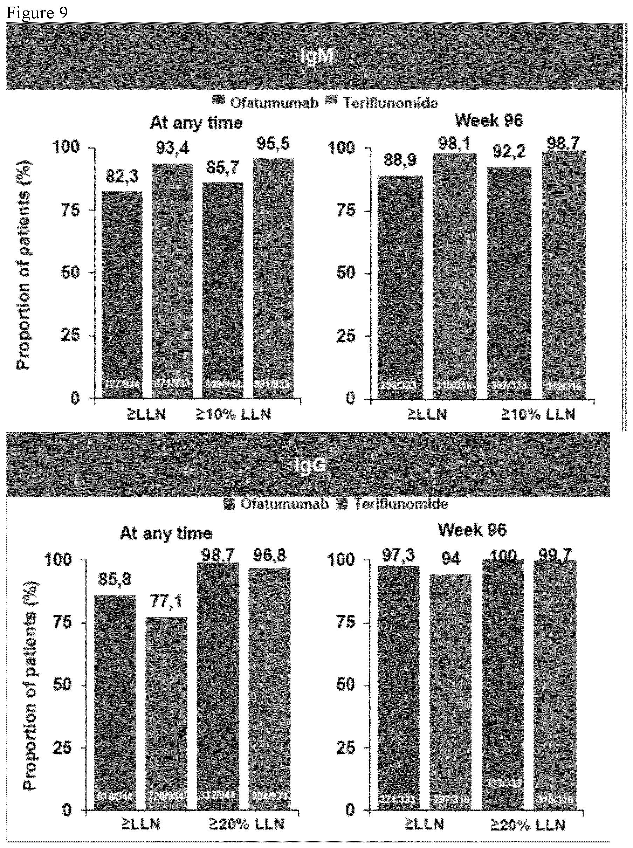

Figure 9 shows the proportion of patients with IgM and IgG levels ≥ LLN, ≥ 10% LLN, and ≥ 20% LLN. At any time post-baseline, about 82-89% of patients on ofatumumab had IgM levels on or above the LLN versus teriflunomide (> 90%) and > 85% of patients on ofatumumab had IgG levels on or above the LLN versus teriflunomide (> 77%).

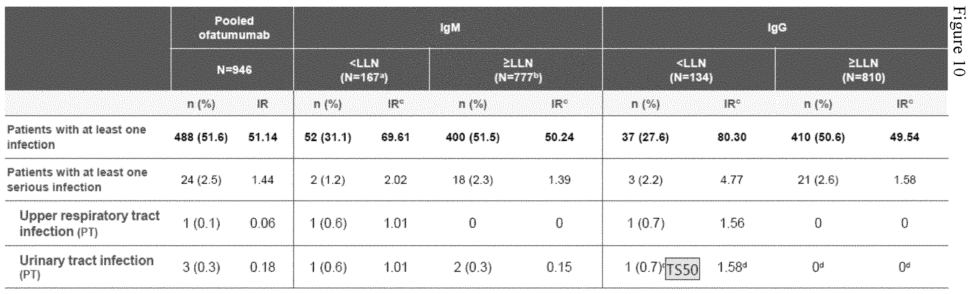

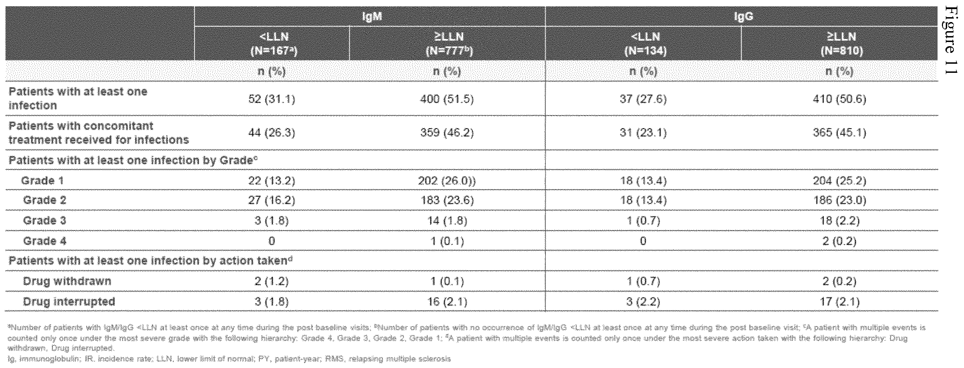

Figures 10 and 11 illustrate the occurrence of serious infections in relation to immunoglobulin levels.
Figure 12 illustrates the change of serum IgG levels from baseline.
Figure 13 illustrates the change of serum IgM levels from baseline.
Figure 14 is a plot of absolute values of IgG parameters by visit-window and 48, 96 and 144 weeks completers in OMB long-term group
Figure 15 is a plot of absolute values of IgG parameters by visit-window and quartiles of baseline values in OMB long-term group
Figure 16 is a plot of absolute values of IgM parameters by visit-window and 48, 96 and 144 weeks completers in OMB long-term group
Figure 17 is a plot of absolute values of IgM parameters by visit-window and quartiles of baseline values in OMB long-term group

### Examples

### Example 1

### Background

Ofatumumab, the first fully-human anti-CD20 monoclonal antibody demonstrated superior efficacy versus teriflunomide in Phase 3 ASCLEPIOS I/II trials, see ECTRIMS Online Library. Hauser S. et al. 09/13/19; 279581; 336. MS patients on ofatumumab had a reduction in annualized relapse rate (ARR) by 50.5% (0.11 vs. 0.22) and 58.5% (0.10 vs. 0.25) compared to Aubagio^{®} (teriflunomide) (both studies p<0.001) in ASCLEPIOS I and II studies respectively. Ofatumumab showed a highly significant suppression of gadolinium (Gd) T1 lesions when compared to Aubagio^{®}, demonstrating a profound suppression of new inflammatory activity. Ofatumumab showed a relative risk reduction of 34.4% in 3-month confirmed disability progression (CDP) (p=0.002) and 32.5% in 6-month CDP (p=0.012) versus Aubagio^{®} in pre-specified pooled analyses.

### Objective

To determine serum immunoglobulin (Ig) levels and investigate association between IgG or IgM levels and new therapeutic options, multiple sclerosis patients were treated with ofatumumab.

### Methods

ASCLEPIOS I and II are double-blind, double-dummy, active comparator-controlled, parallel-group, innovative, adaptive design, multicentre trials. Patients were randomised (1:1) to receive either ofatumumab 20 mg sc injections every 4 weeks (after an initial loading regimen of 20 mg sc doses on Days 1, 7 and 14) or teriflunomide 14 mg orally once daily, for up to 30 months. The studies have flexible durations, with termination occurring in the blinded core treatment epoch according to pre-specified criteria. Patients aged 18-55 years with an Expanded Disability Status Scale (EDSS) score (according to Kurtzke, Neurology. 1983, Nov; 33(11): 1444-52) of 0-5.5 at screening who experienced ≥1 relapse in the past year or ≥2 relapses in the past 2 years or a positive gadolinium-enhancing (Gd+) MRI scan during the year before randomisation were included.

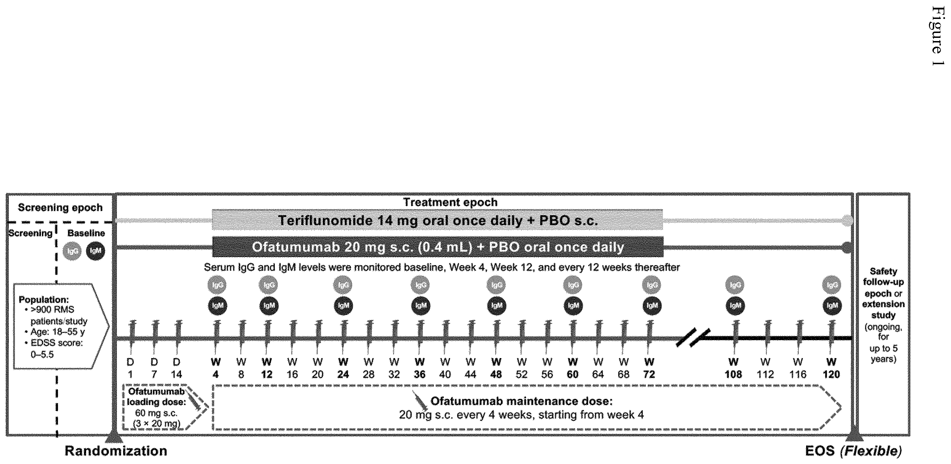

Serum IgG/IgM levels were monitored at baseline, Week (W) 4, W12 and every 12 weeks, see figure 1. Lower limit of normal (LLN) was defined as IgG, 7 g/L and IgM, 0.4 g/L. Outcomes included proportion of patients with notably low IgG/IgM levels and association between notably low IgG/IgM levels and incidence of infections.

### Results

### a) B-cell depletion

Majority of the patients (77.0%) in the ofatumumab group had CD19+ B-cells below LLN, reported as early as Week 1, and proportion of patients with CD19+ B-cell below LLN increased further at Week 2 (95.0%). Depletion was achieved in almost all patients with the initial loading regimen of ofatumumab (3 weekly 20 mg dose at Day 1, 7 and 14).

By Week 12, almost all patients (99.3%) had CD19+ B-cells below LLN and CD19+ B-cell depletion was maintained in >96% patients throughout the treatment period. The median CD19+ B-cell count was zero from Week 2, onwards. In contrast, up to 4.5% patients in the teriflunomide group had B-cells below LLN throughout the study Treatment epoch. The median CD19+ B-cells count was above LLN at all the time points up to Week 120.

| **Visit-window** | **OMB 20 mg N=465 n/M (%)** | **TER 14 mg N=462 n/M (%)** |
|---|---|---|
| Baseline | 5/464 ( 1.1) | 8/ 462 ( 1.7) |
| Week 1 | 338/439 ( 77.0) | 9/443 ( 2.0) |
| Week 2 | 420/442 ( 95.0) | 7/ 437 ( 1.6) |
| Week 4 | 440/450 ( 97.8) | 14/ 444 ( 3.2) |
| Week 12 | 440/443 ( 99.3) | 13/ 439 ( 3.0) |
| Week 24 | 422/429 ( 98.4) | 9/ 419 ( 2.1) |
| Week 36 | 415/ 420 ( 98.8) | 5/408 ( 1.2) |
| Week 48 | 397/404 ( 98.3) | 5/394 ( 1.3) |
| Week 60 | 385/398 ( 96.7) | 8/ 382 ( 2.1) |
| Week 72 | 379/386 ( 98.2) | 3/ 304 ( 1.0) |
| Week 84 | 260/263 ( 98.9) | 4/ 195 ( 2.1) |
| Week 96 | 161/ 162 ( 99.4) | 1/ 121 ( 0.8) |
| Week 108 | 93/ 95 ( 97.9) | 3/67 ( 4.5) |
| Week 120 | 28/28 (100) | 0/ 16 |

### b) Ig Levels

| | | **OMB 20mg N=946** | | |
|---|---|---|---|---|
| **Visit-window** | **Statistics** | **Base** | **Post** | **Change** |
| Immunoglobulin G (g/L), Plasma/Serum | | | | |
| Baseline | n | 945 | | |
| | Mean | 10.178 | | |
| | SD | 2.1663 | | |
| | SE | 0.0705 | | |
| | Min | 5.78 | | |
| | Median | 10.000 | | |
| | Max | 24.08 | | |
| Week 4 | n | 938 | 938 | 938 |
| | Mean | 10.2 | 9.9 | -0.2 |
| Week 12 | n | 925 | 925 | 925 |
| | Mean | 10.190 | 10.060 | -0.130 |
| Week 24 | n | 902 | 902 | 902 |
| | Mean | 10.203 | 9.858 | -0.345 |
| Week 36 | n | 873 | 873 | 873 |
| | Mean | 10.206 | 9.664 | -0.541 |
| Week 48 | n | 846 | 846 | 846 |
| | Mean | 10.211 | 9.765 | -0.446 |
| Week 60 | n | 817 | 817 | 817 |
| | Mean | 10.205 | 9.992 | -0.212 |
| Week 72 | n | 799 | 799 | 799 |
| | Mean | 10.216 | 10.216 | 0.000 |
| Week 84 | n | 584 | 584 | 584 |
| | Mean | 10.234 | 10.312 | 0.078 |
| Week 96 | n | 370 | 370 | 370 |
| | Mean | 10.197 | 10.445 | 0.248 |
| Week 108 | n | 217 | 217 | 217 |
| | Mean | 10.119 | 10.340 | 0.221 |
| Week 120 | n | 96 | 96 | 96 |
| | Mean | 10.2 | 10.5 | 0.3 |

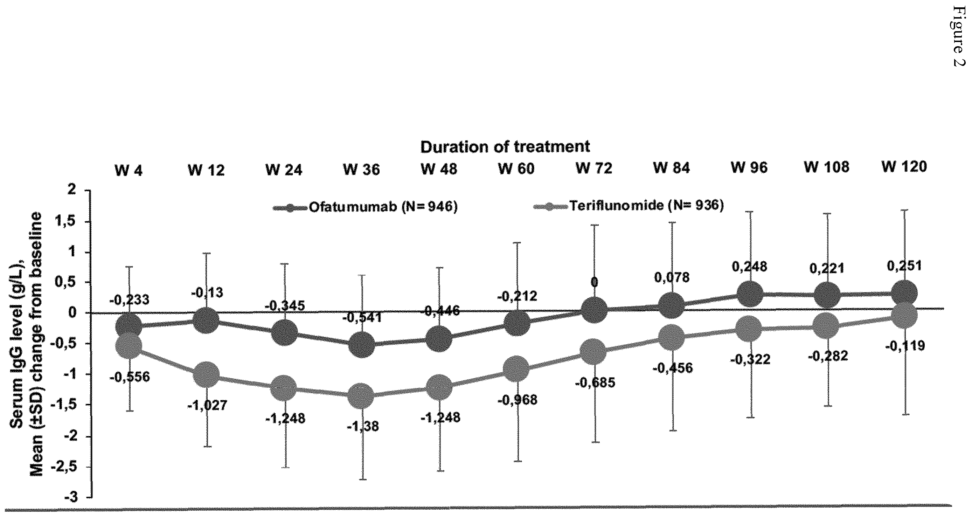

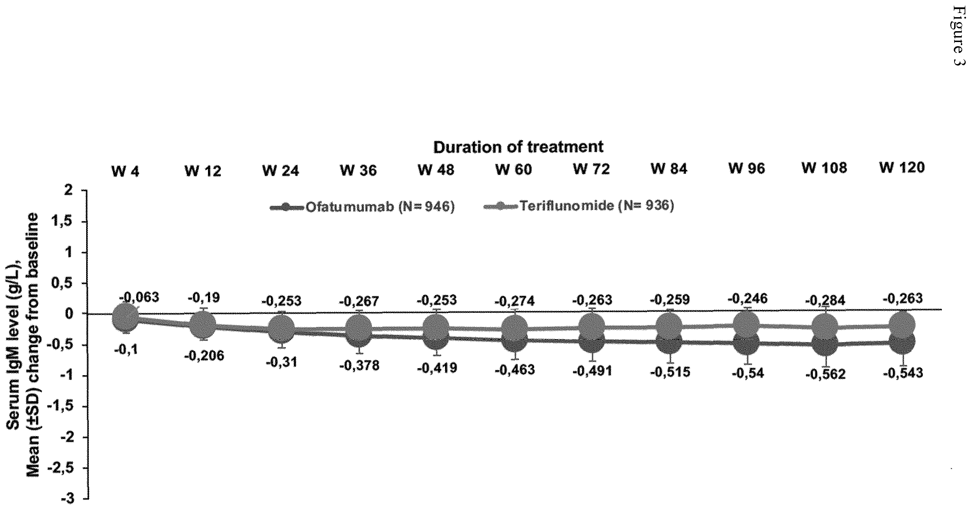

Change in serum IgG levels from baseline is shown in figure 2. Change in serum IgM levels from baseline is shown in figure 3.

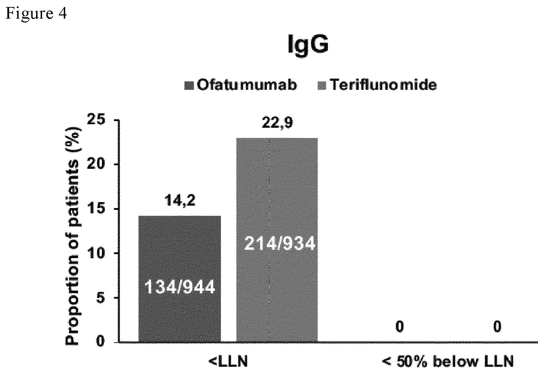

Proportion of patients with Ig levels below LLN is illustrated in figure 4 for IgG.

Infections observed after the first drop of IgG < LLN:
Ofatumumab N=134: 61 (45.5 %)
Teriflunomide N=214: 78 (36.4 %)

### Conclusion

In ofatumumab-treated patients, there is no decrease of IgG levels at week 72 and thereafter. Moreover, there is a turning point at week 36, at which time the trend of decreasing IgG levels reverses, eventually resulting in a net increase in IgG starting around week 72. Hence, a long-term MS therapy without undesirably influencing serum levels of immunoglobulin was provided. The absence or amelioration of negative effects on the Ig, in particular the IgG levels, and thus on the immune system, provides advantages for patients, particular in long-term treatments.

### Example 2

### Methods

In a metanalysis, the results of Example 1 and the results obtained by Derfuss et al. were compared.

### Results

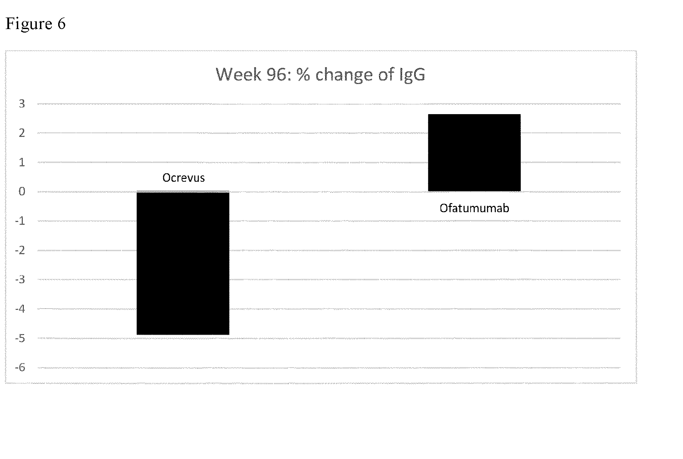

After two years (96 weeks), ocrevus treatment (pooled OPERA, see Fig. 5) has led to a reduction of IgG levels by approx. 5% whereas ofatumumab has led to an increase of about 3%. See Fig. 6.

### Conclusion

Ofatumumab sustains and even increases IgG levels on the long term whereas ocrelizumab leads to a reduction of IgG levels.

### Example 3

### Maintained ofatumumab efficacy in relapsing MS patients who transition from intravenous anti-CD20 therapy

Background: Depletion of B cells in patients with relapsing multiple sclerosis (RMS) using anti-CD20 monoclonal antibodies (mAbs) reduces annualized relapse rates and inflammatory lesion activity on magnetic resonance imaging and delays time to confirmed disability worsening. Anti-CD20 mAbs ocrelizumab and rituximab are administered by intravenous infusion in clinic; ofatumumab is administered subcutaneously with a pre-filled syringe or autoinjector (AI) pen, facilitating self-administration.

Objectives: 12-month, prospective, single-arm, multicenter clinical trial that confirms maintained efficacy of ofatumumab in patients with RMS who transition from intravenous anti-CD20 mAb therapy.

Methods: About 100 adults with RMS are enrolled at 10-20 centers in the USA. Eligible patients have been previously treated with 2-5 consecutive courses of intravenous ocrelizumab or rituximab (other anti-CD20 mAbs are excluded), with a last dose 4-9 months before baseline. Other inclusion criteria are Expanded Disability Status Scale score 5.5 or lower at screening and CD19 B cells depleted to below 1% of anti-CD20 therapy baseline. Patients with suboptimal response to anti-CD20 therapy in the previous 6 months (relapse, ≥2 active gadolinium-enhancing [Gd+] lesions, any new/enlarging T2 lesions, clinical worsening), or who discontinued anti-CD20 therapy because of severe infusion-related reactions or recurrent infections, or with progressive disease, are excluded. All participants receive subcutaneous ofatumumab 20 mg administered by AI pen on Days 1, 7 and 14, then monthly in Months 1-12. The primary endpoint is no change or a reduction in Gd+ lesion count at Month 12. Secondary endpoints are participant retention and changes in immune biomarkers, treatment satisfaction, safety and tolerability at Months 6 and 12. There is a 6-month interim analysis.

Results: The trial complements the ofatumumab phase 3 program in RMS by generating maintained efficacy, retention and satisfaction data based on monthly subcutaneous drug delivery with the AI pen in patients previously treated with ocrelizumab or rituximab.

Conclusions: The trial provides important data on the maintained efficacy of ofatumumab in patients with RMS transitioning from intravenous anti-CD20 therapies.

### Example 4

### Primary Immunodeficiency

The majority of patients with primary antibody deficiencies present with recurrent bacterial infections of the sino-pulmonary tract, including recurrent otitis media, sinusitis, and pneumonia sepsis (Gupta S 2017). In addition, autoimmune manifestations are seen in up to 25% of these patients, with autoimmune hemolytic anemia and autoimmune thrombocytopenia being the most commonly observed deficiency (Goldstein MF et al 2006. Selective IgM immunodeficiency: retrospective analysis of 36 adult patients with review of the literature. Ann Allergy Asthma Immunol. 2006 Dec; 97(6):717-30; Yel L et al 2009. Clinical and immunological features in IgM deficiency. Int Arch Allergy Immunol. 2009; 150(3):291-8.). Primary immunodeficiency should be suspected when recurrent infections are severe, complicated, involving multiple locations, resistant to treatment, caused by atypical organisms or present in family members. In general clinical practice, physicians treating MS patients are aware of the immunosuppressant effect of MS DMTs and risk of MS associated with infections and should therefore evaluate the patient immune status with relevant medical history or findings based on physical examination.

### Secondary Immunoglobulin Deficiency and Risk of Infections

Decline in serum immunoglobulin and risk of infection has been reported in patients with MS treated with anti-CD20 therapies. Rates may vary according to the measuring instrument used, the population setting (e.g. outpatient or inpatient, socio-demographic characteristics) and the design of the study (e.g. immunoglobulin assays, normal values, frequency of monitoring etc.) or method used to show or refute association.

Among 3595 patients treated with rituximab over 11 years with 14,816 PY (Patient-Years) with over 1246 patients had > 5 years of follow-up, 863 (24%) developed IgM < LLN for at least 4 months while 143/3595 (4%) developed IgG < LLN for at least 4 months. For both of these Ig classes, serious infection rates per 100 PY were similar before (IgM 2.80, 95% CI 2.09-3.75; IgG 6.75, 95% CI 4.48-10.15) and during/after development of low Ig (IgM 3.84, 95% CI 3.20-4.62; IgG 8.16, 95% CI 5.86-11.36). Serious infection rates in patients who developed low IgG levels were higher than corresponding rates in patients who never developed low IgG and higher than those in the all-exposure population, both before and after development of low IgG levels, suggesting that these patients may have a higher inherent risk of developing serious infection events (van Vollenhoven RF, Fleischmann RM, Furst DE, Lacey S, Lehane PB. Longterm Safety of Rituximab: Final Report of the Rheumatoid Arthritis Global Clinical Trial Program over 11 Years. J Rheumatol. 2015;42(10):1761-1766. doi:10.3899/jrheum.15005).

Another study of 700 patients treated with rituximab in a single center reviewed severe infection and effects of hypogammaglobulinemia during therapy in Rheumatic and Musculoskeletal Diseases showed similar results on the association of Ig and infection risk. For IgM, the rates of serious infections were similar in patients with low IgM at baseline (10.6/100 PY), patients before acquiring low IgM during treatment (9.8/100 PY), and those with normal IgM (9.2/100 PY) (Md Yusof MY, et al. Predicting Severe Infection and Effects of Hypogammaglobulinemia During Therapy With Rituximab in Rheumatic and Musculoskeletal Diseases. Arthritis Rheumatol. 2019;71(11):1812-1823). However, serious infections rates were higher in patients with low IgG at baseline (16.4/100 PY) and in those who acquired low IgG during or after rituximab (RTX) treatment (21.3/100 PY) versus those with normal IgG (9.7/100 PY). This is supported by a single center retrospective review of 177 patients with multisystem autoimmune disease receiving rituximab between 2002 and 2010. There was no difference in the proportion of patients affected by infection (normal IgM 37% vs low IgM 43%) according IgM levels (Marco H, Smith RM, Jones RB, et al. The effect of rituximab therapy on immunoglobulin levels in patients with multisystem autoimmune disease. BMC Musculoskelet Disord. 2014;15:178. Published 2014 May 25. doi:10.1186/1471-2474-15-178).

However, factors known to be associated with decreased gamma-globulin levels with rituximab in rheumatoid arthritis patients include background chronic corticosteroid use and older age (van Vollenhoven RF, Emery P, Bingham CO 3rd, et al. Longterm safety of patients receiving rituximab in rheumatoid arthritis clinical trials [published correction appears in J Rheumatol. 2010 Oct;37(10):2198]. J Rheumatol. 2010;37(3):558-567). This varies significantly compared to baseline characteristics of multiple sclerosis patients treated with other anti-CD20 therapies. In ocrelizumab treated patients in multiple sclerosis, over 5.5 years of treatment in clinical studies revealed association with infection was noted strongest for IgG (6.50/100PY vs 2.11/100PY) and less so for IgM (3.66/100PY vs 1.88/100PY) (https://onlinelibrary.ectrims-congress. eu/ectrims/2019/stockholm/279399/tobias. derfuss.serum.immunoglobulin .levels.and.risk.of.serious.infecti).

Therefore, it can be assumed that decline in IgG is more clinically meaningful compared to decline in IgM in patients treated with anti-CD20 therapies.

### Pharmacological differences between Kesimpta, Ocrevus and Rituxan

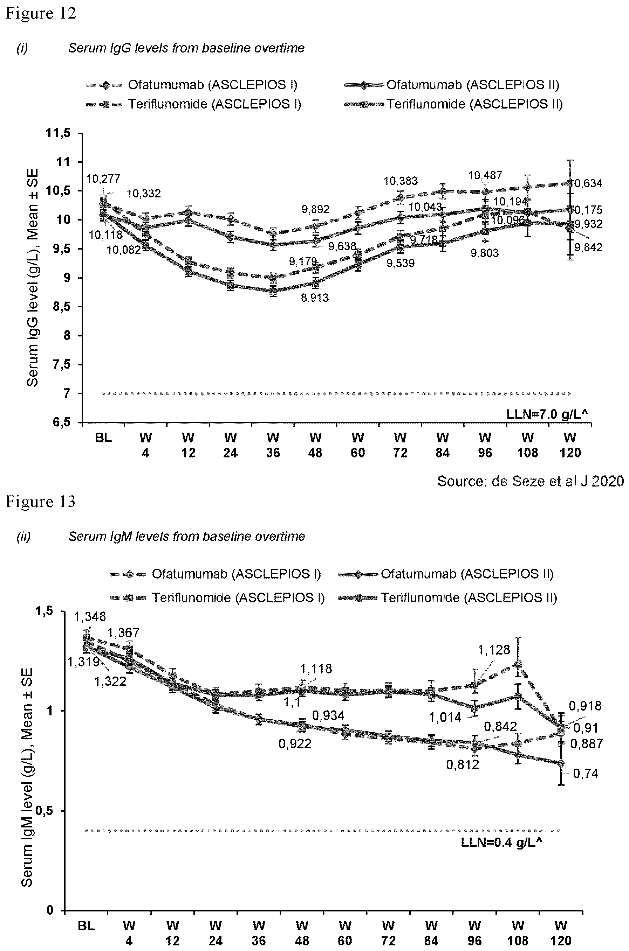

Pharmacologically, ofatumumab has a distinct mechanism of action when compared to other anti-CD20 therapies such as rituximab or ocrelizumab. Although all of these antibodies bind to the CD20 receptor on B-cells, nonclinical data indicate a number of unique properties of ofatumumab. No relevant difference in ADCC activity of ofatumumab compared to ocrelizumab was observed, while rituximab was less active [Summary of clinical pharmacology (SCP)-Figure 12]. In addition, B-cell binding studies showed a lower off-rate (i.e. dissociation rate of the antibody from the CD20 receptor) for ofatumumab in comparison to rituximab, which is of functional importance [Nonclinical Overview].

Ocrelizumab and rituximab are both administered at high doses intravenously, 600 mg and 2000 mg, respectively, and consequently, have to pass the blood circulatory system and organs including the liver and spleen, before they finally reach the lymphoid system. In contrast, ofatumumab administered subcutaneously at a dose of 20 mg, enters the systemic circulation via an indirect route through the lymphatics (Richter et al 2012), which contributes to a lower dose in achieving equivalent clinical efficacy, better tolerability and a lower clinical risk (i.e. no hypo-IgG, no neutropenia, faster repletion of CD20) when compared to a high dose intravenous route of administration. Clinically, ofatumumab differs from other anti-CD20 mAbs in the below measured clinical characteristics:
- As to be expected for a CD20 therapy, treatment with ofatumumab was associated with some level of IgM decline but the majority of the patients remained above LLN. The decline in mean serum IgM was predominantly noted in the first 48 weeks only.
- In contrast to ocrelizumab, an increase of 2.2% (+0.249 g/L) in mean serum IgG value at Week 96 from baseline was observed for the ofatumumab (OMB) treated patients in ASCLEPIOS studies. Whereas in the teriflunomide group, a decrease of 4.1% (-0.421 g/L) in mean IgG value from baseline was observed at Week 96.
- No risk of neutropenia is noted with ofatumumab treatment in MS patients. Mean and median values of neutrophil count remained within the normal ranges and baseline neutrophil counts did not predict any declines over the course of the study based on summary statistics by visit-window in both Study G2301 and Study G2302.
- In Pool C2, after study treatment was discontinued, 28 of 40 patients (70.0%) in the ofatumumab group who reached Week 48 after the last dose date showed B-cell repletion (had B cells on or above LLN or baseline) compared with 22 of 35 patients (62.9%) in the teriflunomide group. In study OMS115102, B cells for all subjects were repleted, with the exception of one subject at approximately 48 weeks post study [OMS115102-Section 8.2.3].
   ∘ As per Ocrevus USPI, in 51 patients from MS clinical studies, the median time for B-cell counts to return to either baseline or LLN was 72 weeks (range 27-175 weeks) after the last OCREVUS infusion and within 2.5 years after the last infusion, B-cell counts rose to either baseline or LLN in 90% of patients.
   ∘ As per Rituxan label, the majority of Rheumatoid Arthritis patients showed peripheral B-cell depletion for at least 6 months. A small proportion of patients (~4%) had prolonged peripheral B-cell depletion lasting more than 3 years after a single course of treatment.

### Serum Immunoglobulin and Ofatumumab in MS Studies

In Pool C2, serum immunoglobulin results demonstrated that mean serum IgM levels remained well within the reference ranges over time (reference range in patients aged >19 years of age: 0.40-2.30 g/L) (Figure 12 and 13). Although a decrease of 30.9% (-0.420 g/L) in mean IgM value was noted from baseline to Week 48 but the slope of decline in mean IgM among Week 96 completers was low (38.8%, -0.537 g/L) compared to first 48 Week completers. In both Week 48 and Week 96 completers, mean serum IgM levels remained well within the reference ranges.

### Immunoglobulin leading to discontinuation

Although, serum IgG and/or serum IgM < LLN were exclusion criteria for Study G2301 and G2302, the protocol recommendation for notably low levels of IgG/M for treatment interruption was defined as a level that is 20% below the LLN for IgG and a level 10% below the LLN for IgM. From a clinical practice point of view, these protocols stipulated thresholds that may be regarded as rather conservative.

Overall, 167 of 944 (17.7%) patients treated with ofatumumab reported IgM below LLN anytime during post baseline. Of the 167 patients, only 32 patients (3.2%) discontinued treatment with ofatumumab, while the majority of the remaining patients continued to receive treatment with ofatumumab in the ongoing clinical studies. Among the 32 patients (3.2%) who discontinued treatment with ofatumumab
- six of 32 patients in ofatumumab group and 2 of 7 patients in teriflunomide group reported an infection post study drug discontinuation. These infections were predominantly upper respiratory tract of urinary tract infections;
- no cases of serious opportunistic infections were reported in the MS clinical studies;
- none of the patients received immunoglobulin substitution therapy for decrease in IgG/M.

In chronic diseases like MS and due to the double-blinded nature of the clinical studies, the treating physicians could not continue study treatment interruptions per protocol to avoid any risk of disease activity and potential relapse. Therefore, the discontinuation rates could have been triggered by the protocol stipulated threshold and to initiate alternate treatment for MS disease activity.

### Immunoglobulins and Infection Risk

Table 2.1 below provides an overview over a proportion of patients with IgM/IgG levels below the lower limit of normal (<LLN [g/L]: IgM, 0.4; IgG, 7.0), and association of IgM/IgG levels with incidence of infections that occurred up to one month prior and one month after any decrease in IgM/IgG levels (<LLN vs ≥LLN) was analyzed.

Overall, 167 of 944 (17.7%) treated with ofatumumab reported IgM below LLN anytime during post baseline. Of the 167 patients
- 124 (74.3%) with at least one episode of IgM<LLN patients temporarily interrupted ofatumumab treatment per protocol;
- the majority of the patients continued to receive treatment with ofatumumab in the ongoing clinical studies and did not discontinue study treatment;
- overall incidence of infections was lower in patients with a decrease in IgM <LLN (52 patients 31.1%) compared to patients who maintained IgM >LLN (400 patients, 51.5%);
- the pattern of serious infections was also consistent with overall infections, with higher incidence of serious infections noted in patients who maintained IgM > LLN (18 patients, 2.3%) compared to patients with an infection 1 month prior to until 1 month after a decrease in IgM < LLN (2 patients, 1.2%). Both the serious infections (upper respiratory tract infection, urinary tract infection) in the IgM < LLN group were not opportunistic in nature and were typically expected in MS patients;
- most frequently reported infection adverse events (AE, ≥2%) were upper respiratory tract infection, urinary tract infection and oral herpes, which were consistent with overall patients with IgM ≥LLN and overall MS patient population;
- consistent with patients with IgM≥LLN, the majority of infections in patients in the IgM<LLN group were mild to moderate, patients recovered with standard of care and infections were not treatment limiting.

There was no decrease in mean values over time compared to baseline for serum IgG. The proportion of patients with IgG below LLN anytime during post baseline visit was less in the ofatumumab group (14.2%; 134/944) versus the teriflunomide group (22.9%; 214/934). Among the Week-96 completers, no patients were reported with the protocol-stipulated decline of immunoglobulin G levels below 20% LLN [SCS Appendix 1Table C2 3.53] [SCS Appendix 1-Table C2 3.5-3a].

Overall, there were no cases of PML or Hepatitis B reactivation, nor any serious potential OI such as disseminated zoster, identified in any MS clinical trial with ofatumumab. Review of data showed no increased incidence of malignancy with ofatumumab treatment. Whereas the risk of malignancy remains potential as for any immunomodulator, the data do not indicate an increased risk for any malignancy.

**Table 0-1 Summary of IgG related AEs leading to interruptions and discontinuations and treatment emergent Infections, started 1 month prior to 1 month after any decline of IgG or IgM on the subset of patients who had IgG/M <LLN vs IgG/M > = LLN**

| **Criterion** | **IgM <LLN N=167 n (%)** | **IgM ≥LLN N=777 n (%)** | **IgG <LLN N=134 n (%)** | **IgG ≥LLN N=810 n (%)** | |
|---|---|---|---|---|---|
| Number of patients at least one episode of Ig decline | 167 (100%) | NA | 134 (100%) | NA | |
| Number of patients with treatment interruptions with Ig decline | 124 (74.3%) | NA | 2 (1.5%) | NA | |
| Number of patients with treatment discontinuations with Ig decline | 30 (1.8%) | NA | 4 (3.0%) | NA | |

| **Criterion** | | **IgM** <LLN N=167 n (%) | **IgM ≥LLN** N=777 n (%) | **IgG** <LLN N=134 n (%) | **IgG ≥LLN** N=810 n (%) |
|---|---|---|---|---|---|
| Number of patients with at least one infection AE with Ig decline | | 52 (31.1%) | 400 (51.5%) | 37 (27.6%) | 410 (50.6%) |
| Number of patients with at least 1 infection AE by Grade | | 22 (13.2%) | 202 (26.0%) | 18 (13.4%) | 204 (25.2%) |
| | Grade 1 | | | | |
| | Grade 2 | 27 (16.2%) | 183 (23.6%) | 18 (13.4%) | 186 (23.0%) |
| | Grade 3 | | | | |
| | Grade 4 | 3 (1.8%) | 14 (1.8%) | 1 (0.7%) | 18 (2.2%) |
| | | 0 | 1 (0.1%) | 0 | 2 (0.2%) |
| Number of patients with at least one infection AE by outcome | | 3 (1.8%) | 12 (1.5%) | 1 (0.7%) | 13 (1.6%) |
| | Not recovered/not resolved | 47 (28.1%) | 383 (49.3%) | 36 (26.9%) | 391 (48.3%) |
| | Recovered/resolved | | | | |
| | Recovering/resolving | 2 (1.2%) | 2 (0.3%) | 0 | 3 (0.4%) |
| | Recovered/resolved with sequelae | 0 | 3 (0.4%) | 0 | 2 (0.2%) |
| | | 0 | 0 | 0 | 0 |
| | Fatal | 0 | 0 | 0 | 1 (0.1%) |
| | Unknown | | | | |
| Number of patients with at least one infection SAE | | 2 (1.2) | 18 (2.3) | 3 (2.2) | 21 (2.6) |
| | | 1 (0.6) | - | 1 (0.7%) | - |
| | Upper respiratory tract infection | 1 (0.6) | - | 1 (0.7%) | - |
| | Urinary tract infections | | | | |
| Number of patients with treatment received for infections | | 44 (26.3%) | 359 (46.2%) | 31 (23.1%) | 365 (45.1%) |
| Risk name: Upper Respiratory Tract | | 31 (18.6) | 310 (39.9) | 26 (19.4) | 307 (37.9) |
| Infections | | 14 (8.4) | | 12 (9.0) | |
| | Nasopharyngitis | 10 (6.0) | 140 | 8 (6.0) | 143 |
| | Upper respiratory tract | 4 (2.4) | (18.0) | 2 (1.5) | (17.7) |
| | infection | 4 (2.4) | 74 (9.5) | 2 (1.5) | 77 (9.5) |
| | Pharyngitis | 3 (1.8) | 21 (2.7) | 3 (2.2) | 24 (3.0) |
| | Sinusitis | 2 (1.2) | 24 (3.1) | 3 (2.2) | 21 (2.6) |
| | Influenza | | 55 (7.1) | | 53 (6.5) |
| | Rhinitis | | 20 (2.6) | | 20 (2.5) |
| Risk name: | Urinary tract infection | 12 (7.2) | 95 (12.2) | 9 (6.7) | 95 (12.2) |
| | Urinary tract infection | 11 (6.6) | 81 (10.4) | 9 (6.7) | 81 (10.4) |
| Risk name: | Herpes viral infections | 5 (3.0) | 40 (5.1) | 4 (3.0) | 42 (5.2) |
| | Oral herpes | 2 (1.2) | 22 (2.8) | 2 (1.5) | 23 (2.8) |
| Source: T040 Table C2 5-1.2; T040 Table C2 5-2.1 | | | | | |

### Summary

Kesimpta (Ofatumumab) might be considered under the overarching class of B-cell depleting antibodies. Although comprehensive analysis of data from preclinical safety and clinical trial data including immunoglobulins and infection AEs revealed that ofatumumab showed a distinct mechanism and clinical effects compared to other anti-CD20 therapies based on:
- Low-dose subcutaneous administration;
- No decrease in mean serum IgG over 96 weeks treatment compared to baseline;
- Mean serum IgM levels remained well within the reference ranges over time;
- Infections noted with IgM decline were similar to those patients without any decline in immunoglobulins, i.e. mostly non-serious, grade 1 or 2, and were not treatment limiting;
- No cytopenia was noted during ofatumumab therapy.

As is seen from the section on immunoglobulins, Kesimpta, given as low subcutaneous doses on a monthly schedule, is different from other CD20 antibodies given as intravenous regimens at high doses every 6 months.

These key differences make it a positive outlier in terms of impact to immunoglobulin levels.

### Example 5: Long term Study

Patients were subjected to a long term treatment. Patients received ofatumumab 20 mg sc injections every 4 weeks (after an initial loading regimen of 20 mg sc doses on weeks 0, 1 and 2).

Duration of exposure was as follows:

| **Duration of exposure** | | **Long-term N=1026** | **Newly-switched N=677** | **Overall N=1703** |
|---|---|---|---|---|
| Any exposure | | 1026 (100 ) | 677 (100 ) | 1703 (100 ) |
| Cumulative exposure - n (%) | | | | |
| | >= 1 week | 1026 (100 ) | 677 (100) | 1703 (100 ) |
| | >= 2 weeks | 1026 (100 ) | 677 (100 ) | 1703 (100 ) |
| | >= 3 weeks | 1026 (100 ) | 677 (100 ) | 1703 (100 ) |
| | >= 4 weeks | 1026 (100 ) | 677 (100 ) | 1703 (100 ) |
| | >= 8 weeks | 1026 (100 ) | 671 ( 99.1) | 1697 ( 99.6) |
| | >= 12 weeks | 1026 (100 ) | 668 ( 98.7) | 1694 ( 99.5) |
| | >= 24 weeks | 1019 ( 99.3) | 649 ( 95.9) | 1668 (97.9) |
| | >= 36 weeks | 1016 ( 99.0) | 635 ( 93.8) | 1651 (96.9) |
| | >= 48 weeks | 1013 ( 98.7) | 614 ( 90.7) | 1627 ( 95.5) |
| | >= 60 weeks | 1003 ( 97.8) | 533 ( 78.7) | 1536 ( 90.2) |
| | >= 72 weeks | 989 ( 96.4) | 348 ( 51.4) | 1337 ( 78.5) |
| | >= 84 weeks | 878 ( 85.6) | 10 ( 1.5) | 888 ( 52.1) |
| | >= 96 weeks | 753 ( 73.4) | 0 | 753 (44.2) |
| | >= 108 weeks | 679 ( 66.2) | 0 | 679 ( 39.9) |
| | >= 120 weeks | 652 ( 63.5) | 0 | 652 ( 38.3) |
| | >= 132 weeks | 611 ( 59.6) | 0 | 611 ( 35.9) |
| | >= 144 weeks | 518 ( 50.5) | 0 | 518 (30.4) |
| | >= 156 weeks | 351 ( 34.2) | 0 | 351 ( 20.6) |
| | >= 168 weeks | 231 ( 22.5) | 0 | 231 ( 13.6) |
| | >= 180 weeks | 122 ( 11.9) | 0 | 122 ( 7.2) |
| | >= 192 weeks | 53 ( 5.2) | 0 | 53 ( 3.1) |
| | >= 204 weeks | 6 ( 0.6) | 0 | 6 ( 0.4) |
| | >= 216 weeks | 1 ( 0.1) | 0 | 1 ( 0.1) |
| | < 48 weeks (1 year) | 13 ( 1.3) | 63 ( 9.3) | 76 ( 4.5) |
| | 48 weeks to 96 weeks (1 to 2 years) | 260 ( 25.3) | 614 ( 90.7) | 874 (51.3) |
| | 96 weeks to 144 weeks (2 to 3 years) | 235 ( 22.9) | 0 | 235 ( 13.8) |
| | 144 weeks to 192 weeks (3 to 4 years) | 466 ( 45.4) | 0 | 466 ( 27.4) |
| | > 192 weeks (4 years) | 52 ( 5.1) | 0 | 52 ( 3.1) |
| Exposure in months | | | | |
| | N | 1026 | 677 | 1703 |
| | Mean | 30.9 | 15.3 | 24.7 |
| | SD | 9.26 | 3.61 | 10.72 |
| | Minimum | 3.7 | 1.0 | 1.0 |
| | Q1 | 21.8 | 14.1 | 16.9 |
| | Median | 33.2 | 16.6 | 20.4 |
| | Q3 | 38.1 | 17.6 | 35.0 |
| | Maximum | 50.5 | 20.2 | 50.5 |
| Patient-years | | 2637.7 | 863.0 | 3500.7 |

Demographics were as follows:

| **Characteristic** | | **Long-term N=1026** | **Newly-switched N=676** | **Overall N=1702** |
|---|---|---|---|---|
| Age group - n (%) | | | | |
| | 18 to 30 years | 245 ( 23.9) | 116 ( 17.2) | 361 ( 21.2) |
| | 31 to 40 years | 378 ( 36.8) | 239 ( 35.4) | 617 ( 36.3) |
| | 41 to 55 years | 400 ( 39.0) | 287 ( 42.5) | 687 ( 40.4) |
| | > 55 years | 3 ( 0.3) | 34 ( 5.0) | 37 ( 2.2) |

### Results:

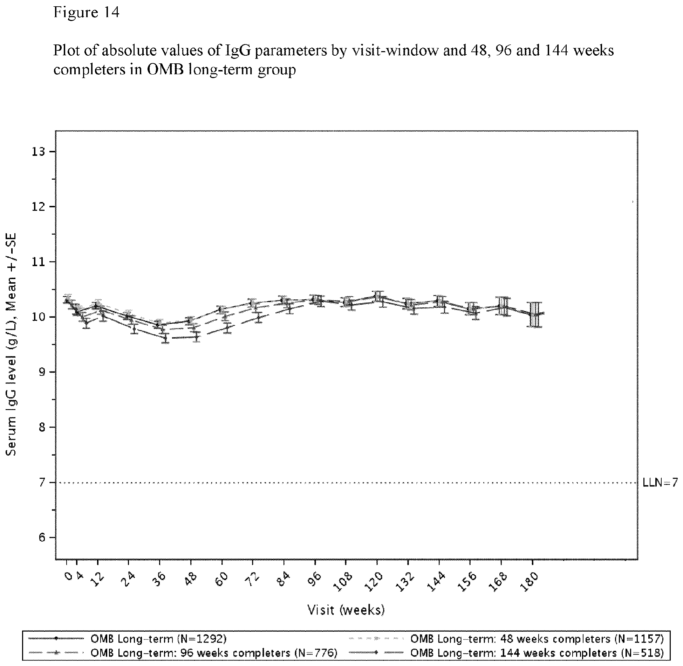

The results on IgG and IgM levels were shown in Figures 14-17.

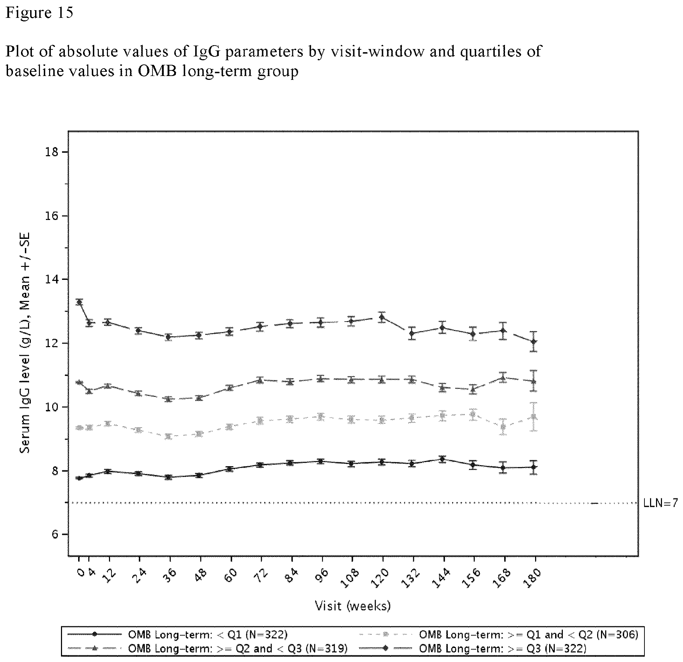

Figure 14 shows that IgG levels were stable over the long-term follow-up of > 4 years. This is of particular importance for the lower quartile, see figure 15.

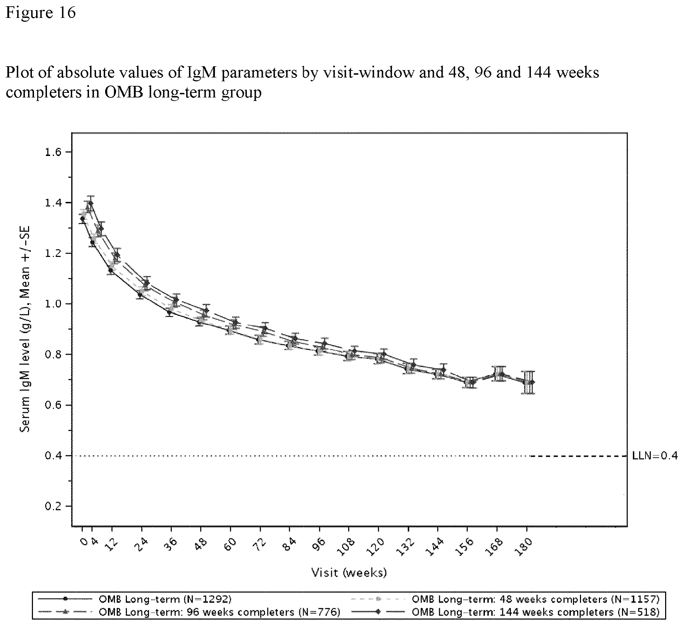

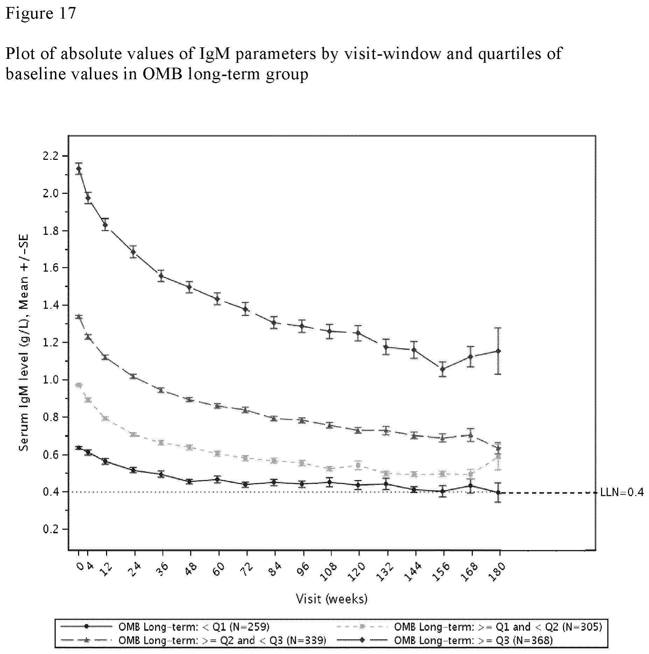

A reduction in IgM levels were seen with longer duration; however, levels remained above the lower limit of normal, see figures 16 and 17.

### Example 6: Characteristics and Outcome of COVID-19 in Patients with Relapsing Multiple Sclerosis Receiving Ofatumumab

Objective: To report clinical characteristics of COVID-19 infection in people with MS (pwMS) receiving ofatumumab 20 mg subcutaneously every 4 weeks

### Methods:

Confirmed or suspected cases of COVID-19 infection in patients receiving ofatumumab in the open-label extension study ALITHIOS were reviewed (data cutoff: December 21, 2020)
COVID-19 cases were classified as confirmed if SARS-CoV2 positive test results were available, or patient was reported to be diagnosed with COVID-19
Suspected cases of COVID-19 were classified as suspected if without a positive SARS-CoV2 test or definitive diagnosis
The following COVID-19 case characteristics were assessed:
Patient demographics
COVID-19 seriousness category*
Ofatumumab treatment duration and action taken with ofatumumab (treatment interruption)
Interventions and COVID-19 outcomes
*Serious criteria is based on the regulatory reporting definition established by ICH, for the purposes of regulatory reporting obligations, and consists of fatal, life-threatening, hospitalization and medically significant
Patient characteristics: Overall population
Patient demographics and drug exposure are shown in the table below:
45% (466/1026) of patients in the long-term group have drug exposure of 3-4 years Over 90% (614/677) of patients in the newly switched group have ofatumumab exposure of 1-2 years

| Characteristics | | Ofatumumab- long term group N=1026 | Ofatumumab - newly switched N=677* | Overall N=1703* |
|---|---|---|---|---|
| Age, mean, years | | 38 | 40 | 39 |
| Age group, % of patients | | | | |
| | 18 - 30 | 24 | 17 | 21 |
| | 31-40 | 37 | 35 | 36 |
| | 41 - 55 | 39 | 43 | 41 |
| | >55 | 0.3 | 5 | 2 |
| Sex, n (%) | | | | |
| | Male | 296 (29) | 220 (33) | 516 (30) |
| | Female | 730 (71) | 456 (68) | 1186 (70) |
| Exposure, months | | | | |
| | Mean | 30.9 | 15.3 | 24.7 |
| | Range | 3.7 - 50.5 | 1.0 - 20.2 | 1.0 - 50.5 |
| | Patient-years | 2637.7 | 863.0 | 3500.7 |

### Results: COVID-19 cases overview

As of 21 December 2020, 35 out of 1703 patients had a confirmed COVID-19 infection and/or COVID-19 pneumonia in the ongoing open-label, extension ALITHIOS clinical trial for ofatumumab (Table)
All the non-serious cases were reported as completely recovered
For 21 cases no change in ofatumumab treatment required and for 5 cases treatment was temporarily interrupted due to confirmed COVID-19 infection Of the 6 serious cases, 5 had complete recovery and in one patient the COVID-19 outcome was fatal (details as below).
The 48 year-old patient, with no associated risk factors* (*relevant comorbidities, such as chronic lung condition, diabetes, hypertension or malignancy), reported symptoms of COVID-19 (pneumonia, fever, weakness, cough and dyspnoea) after approximately 3 years and 7 months of ofatumumab treatment. The patient was hospitalized and received steroids, antivirals, antibiotics and COVID-19 convalescent plasma. The COVID-19 outcome was reported as not related to ofatumumab treatment.

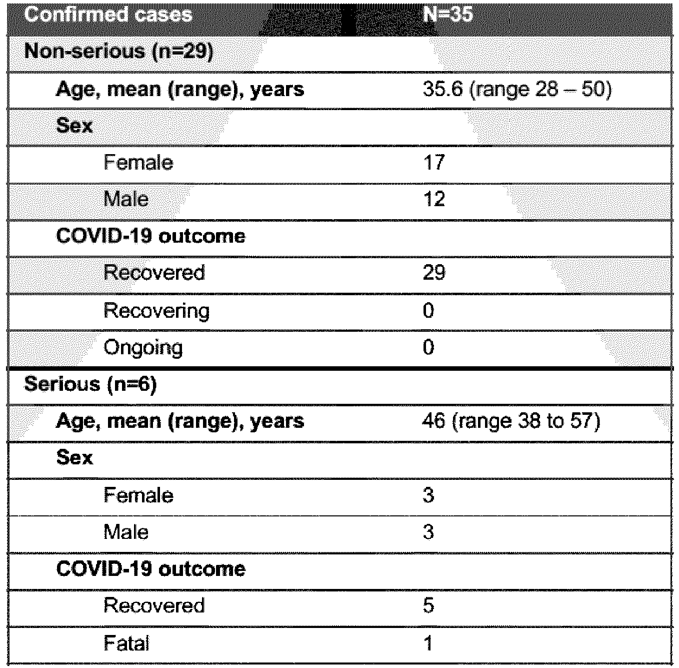

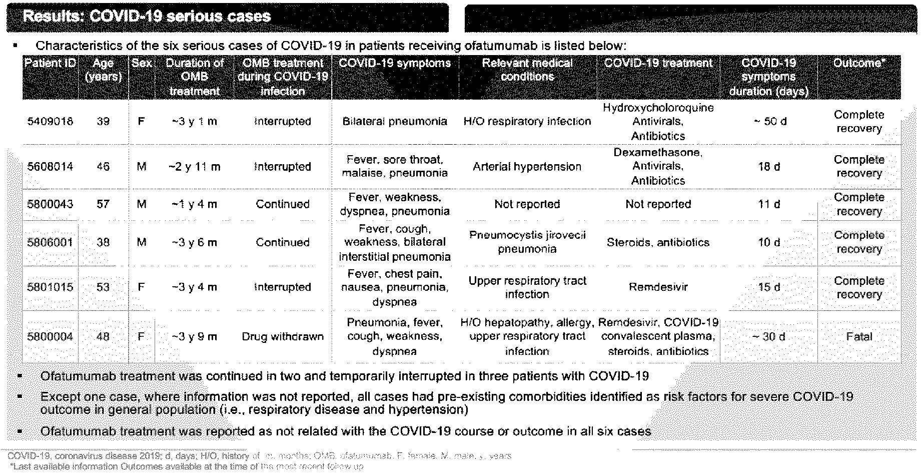

### Conclusions:

Of the total 1703 patients in the ongoing ALITHIOS study, 35 confirmed cases of COVID-19 infection and/or COVID-19 pneumonia have been reported. Complete recovery in 34 cases; one case had fatal outcome. None of the cases were suspected because of ofatumumab treatment.

Based on the review of reported cases, the clinical presentations and outcomes of COVID-19 cases in pwMS on ofatumumab therapy was similar to other reports on MS population (Richadson S, et al. JAMA. 2020;323:2052-2059; Sormani MP, et al. Lancet Neurol. 2020 Jun; 19(6):481-482; Montero-Escribano P, et al. Mult Scler Relat Disord. 2020;42:102185; Safavi F, et al. Mult Scler Relat Disord. 2020;43:102195; Barzegar M, et al. Mult Scler Relat Disord. 2020;45:102276) and in the general population (Bchetnia M, et al. J Infect Public Health. 2020;13(11):1601-1610).

New patients can initiate therapy with ofatumumab in accordance with local guidelines on testing for SARS-CoV2

The invention further provides the following non-limiting embodiments:
1. B cell and/or T cell inhibitor for use in the treatment of multiple sclerosis, said treatment comprising
   (a) optionally administering a B cell and/or T cell inhibitor other than ofatumumab,
   (b) monitoring serum IgG level,
   (c) selecting ofatumumab as B cell and/or T cell inhibitor if serum IgG level decreases.
2. B cell and/or T cell inhibitor for use according to embodiment 1, wherein in step (c) ofatumumab is selected as B cell and/or T cell inhibitor if serum IgG level falls below the lower limit of normal.
3. B cell and/or T cell inhibitor for use according to embodiment 1, wherein in step (c) ofatumumab is selected as B cell and/or T cell inhibitor if serum IgG level falls below a concentration of 900 mg/dl.
4. B cell and/or T cell inhibitor for use according to embodiment 1, wherein in step (c) ofatumumab is selected as B cell and/or T cell inhibitor if serum IgG level falls below 80% of the baseline level at the start of therapy.
5. B cell and/or T cell inhibitor for use in the treatment of multiple sclerosis, said treatment comprising
   (a) optionally administering B cell and/or T cell inhibitor other than ofatumumab,
   (b) assessing the predisposition of the patient for an increased risk of infections,
   (c) selecting ofatumumab as B cell and/or T cell inhibitor if a predisposition for an increased risk of infections is identified.
6. B cell and/or T cell inhibitor for use according to embodiment 5, wherein the predisposition for bacterial infections, fungal infections, viral infections are assessed.
7. B cell and/or T cell inhibitor for use according to any one of the preceding embodiments, wherein the B cell and/or T cell inhibitor in step (a) is ocrelizumab or rituximab.
8. B cell and/or T cell inhibitor for use according to any one of the preceding embodiments, wherein the monitoring in step (b) is carried out every 1 - 12 months.
9. Ofatumumab for use in the treatment of multiple sclerosis, wherein the treatment is a long-term treatment and wherein serum IgG level is maintained within a range, wherein said range is essentially the same in untreated patients.
10. Ofatumumab for use in the treatment according to embodiment 9, wherein the serum IgG level is within a range of from 500 to 1800 mg/dl.
11. Ofatumumab for use in the treatment of multiple sclerosis, wherein patients having a lowered serum IgG level are treated.
12. Ofatumumab for use in the treatment according to embodiments 11, wherein the lowered serum IgG level at the start of treatment is below 900 mg/dl.
13. Ofatumumab for use in the treatment according to embodiments 11 or 12, wherein the lowered serum IgG level at the start of treatment is below the lower limit of normal.
14. Ofatumumab for use in the treatment according to embodiments 11 to 13, wherein the change in serum IgM levels from baseline is less than 100 mg/dl.
15. Ofatumumab for use in the treatment of multiple sclerosis, wherein patients are treated having risk factors associated with serum Ig levels, in particular serum IgG levels.
16. Ofatumumab for use in the treatment according to embodiment 15, wherein the risk factors are metabolic abnormalities, in particular obesity and metabolic syndrome.
17. Ofatumumab for use in the treatment according to embodiment 15, wherein the risk factors are habits, in particular alcohol consumption and/or smoking.
18. Ofatumumab for use in the treatment according to embodiment 15, wherein the risk factors are malignancies.
19. Ofatumumab for use in the treatment according to embodiment 18, wherein patients are treated
   with known risk factors for malignancies,
   who are being actively monitored for recurrence of malignancy and/or
   with a known active malignancy.
20. Ofatumumab for use in the treatment according to embodiments 11 to 19, wherein the treatment is a long-term treatment.
21. B cell and/or T cell inhibitor or ofatumumab for use according to any one of the preceding embodiments, wherein ofatumumab is administered at a dose of 10 to 30 mg every 4 weeks, preferably 20 mg every 4 weeks.
22. B cell and/or T cell inhibitor or ofatumumab for use according to any one of the preceding embodiments, wherein ofatumumab is administered subcutaneously.
23. B cell and/or T cell inhibitor or ofatumumab for use according to any one of the preceding embodiments, wherein ofatumumab is administered with a loading dose.
24. B cell and/or T cell inhibitor or ofatumumab for use according to embodiment 23, wherein 20 mg ofatumumab at weeks 0, 1 and 2 is administered as a loading dose.
25. B cell and/or T cell inhibitor or ofatumumab for use according to any one of the preceding embodiments, wherein multiple sclerosis is selected from relapsing multiple sclerosis, in particular clinically isolated syndrome (CIS), relapsing remitting multiple sclerosis (RRMS) and secondary progressive multiple sclerosis (SPMS).
26. B cell and/or T cell inhibitor or ofatumumab for use according to any one of the preceding embodiments, wherein multiple sclerosis is selected from primary progressive multiple sclerosis (PPMS) or progressive relapsing multiple sclerosis (PRMS).
27. B cell and/or T cell inhibitor or ofatumumab for use according to any one of the preceding embodiments, wherein a premedication is administered to the patient before the first dose of ofatumumab is administered.
28. B cell and/or T cell inhibitor or ofatumumab for use according to embodiment 27, wherein the premedication comprises acetaminophen, antihistamines and/or steroids.
29. B cell and/or T cell inhibitor or ofatumumab for use according to embodiment 27 or 28, wherein the premedication is administered 30 to 60 minutes prior to an ofatumumab injection.
30. B cell and/or T cell inhibitor or ofatumumab for use according to any one of embodiments 1 to 26, wherein no premedication is administered prior to the first dose of ofatumumab.
31. B cell and/or T cell inhibitor or ofatumumab for use according to any one of the preceding embodiments, wherein a patient acutely or previously infected by COVID-19 is treated.
32. B cell and/or T cell inhibitor or ofatumumab for use according to any one of the preceding embodiments, wherein the treatment is continued during COVID-19 infection.
33. B cell and/or T cell inhibitor or ofatumumab for use according to any one of embodiments 1 to 31, wherein the treatment is interrupted during COVID-19 infection und continued after overcoming the infection.

## Claims

1. Ofatumumab for use in both treating multiple sclerosis in a patient and obtaining an increase in the level of serum IgG in said patient.

2. Ofatumumab for use according to claim 1, wherein the patient has a predisposition for an increased risk of infections.

3. Ofatumumab for use according to claim 1 or claim 2, wherein the patient has a risk factor associated with serum IgG levels, optionally wherein the risk factor is obesity, metabolic syndrome, alcohol consumption, or smoking.

4. Ofatumumab for use according to any one of the preceding claims, wherein the increase is relative to the level of serum IgG in the patient at the start of the treatment with ofatumumab.

5. Ofatumumab for use according to any one of the preceding claims, wherein the patient has a serum IgG level below 900 mg/dl at the start of the treatment with ofatumumab.

6. Ofatumumab for use according to any one of the preceding claims, wherein an increase in the level of serum IgG is obtained in the patient after 72 weeks of treatment with ofatumumab, optionally wherein an increase is obtained at week 96 of the treatment.

7. Ofatumumab for use according to any one of the preceding claims, wherein the multiple sclerosis is relapsing multiple sclerosis (RMS), relapsing remitting multiple sclerosis (RRMS), secondary progressive multiple sclerosis (SPMS), primary progressive multiple sclerosis (PPMS) or progressive relapsing multiple sclerosis (PRMS).

8. Ofatumumab for use according to any one of the preceding claims, wherein ofatumumab is administered subcutaneously.

9. Ofatumumab for use according to any one of the preceding claims, wherein ofatumumab is administered at a dose of 10 to 30 mg monthly, preferably at a dose of 20 mg monthly, optionally wherein, prior to administering the dose of ofatumumab monthly, ofatumumab is administered at a dose of 20 mg at weeks 0, 1 and 2 of the treatment.
